(19) Europäisches Patentamt
European Patent Office
Office européen des brevets

(11) **EP 1 923 040 A2**

(12) **DEMANDE DE BREVET EUROPEEN**

(43) Date de publication:
**21.05.2008 Bulletin 2008/21**

(21) Numéro de dépôt: **07120911.8**

(22) Date de dépôt: **16.11.2007**

(51) Int Cl.:
***A61K 8/19*** *(2006.01)* ***A61K 8/26*** *(2006.01)*
***A61K 8/29*** *(2006.01)* ***A61K 8/49*** *(2006.01)*
***A61Q 1/02*** *(2006.01)* ***A61Q 1/06*** *(2006.01)*
***A61Q 1/10*** *(2006.01)* ***A61Q 1/12*** *(2006.01)*
***A61Q 3/02*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité:
**17.11.2006 FR 0654982**
**17.11.2006 FR 0654979**
**17.11.2006 FR 0654980**
**17.11.2006 FR 0654981**
**17.11.2006 FR 0654977**
**17.11.2006 FR 0654975**
**12.12.2006 FR 0655459**
**12.12.2006 FR 0655457**
**12.12.2006 FR 0655458**
**12.12.2006 FR 0655460**
**12.12.2006 FR 0655454**
**12.12.2006 FR 0655452**

(71) Demandeur: **L'ORÉAL**
**75008 Paris (FR)**

(72) Inventeur: **THEVENET, Ludovic**
**92340, BOURG LA REINE (FR)**

(74) Mandataire: **Tanty, François**
**Nony & Associés,**
**3, rue de Penthièvre**
**75008 Paris (FR)**

(54) **Composition cosmétique comportant un pigment interferentiel et un agent de coloration sensible à un stimulus extérieur**

(57) La présente invention concerne une composition cosmétique comportant, dans un milieu cosmétiquement acceptable, au moins un pigment interférentiel multicouche et au moins l'un d'un agent de coloration sensible à au moins un stimulus extérieur, d'un pigment réfléchissant à reflet métallique et d'un pigment diffractant, la couvrance de la composition étant supérieure ou égale à 25, mieux 30, le pigment interférentiel multicouche étant en une teneur conférant à la composition un écart de couleur ΔE supérieur ou égale à 5 entre la couleur dans la masse et la couleur après application.

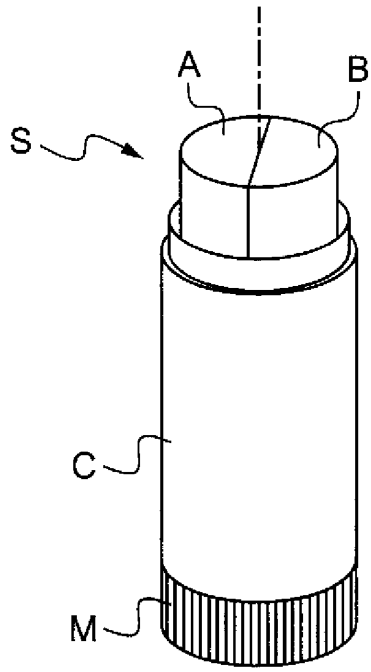


Fig.2

EP 1 923 040 A2

## Description

La présente invention concerne les compositions cosmétiques et plus particulièrement les compositions de maquillage de la peau, des lèvres ou des phanères.

## Arrière-plan

Il est connu d'incorporer dans des compositions de maquillage des agents de coloration dont la couleur est sensible à un stimulus extérieur, ces agents de coloration étant encore appelés agents de coloration « Xchromes », X désignant le facteur extérieur. Il peut s'agir par exemple d'agents de coloration photochromes, thermochromes, solvatochromes ou tribochromes.

Il est connu d'incorporer dans des compositions de maquillage des pigments diffractants afin d'apporter un aspect irisé ou un effet de décomposition de la lumière qui induit un changement de couleur en fonction de l'angle d'incidence de la lumière ou d'observation.

Il est connu d'introduire dans les compositions de maquillage des pigments diffusants pour produire des couleurs par un phénomène d'absorption de la lumière par des chromophores spécifiques.

A ces pigments diffusants, qui sont nécessaires pour bénéficier d'un fond coloré continu et suffisamment couvrant, peuvent être ajoutées des particules à effet(s) pour créer par exemple des points de brillance ou conférer un aspect nacré.

Toutefois, l'intensité de la couleur produite par ces compositions peut s'avérer insuffisante pour conduire à un résultat complètement satisfaisant.

Les pigments interférentiels multicouche, comportant un empilement de couches dont les indices de réfraction et les épaisseurs sont convenablement choisis afin de générer une couleur par un phénomène d'interférences, permettent de produire une intensité de couleur plus importante que les pigments diffusants ci-dessus.

A la connaissance de l'inventeur, dans les compositions disponibles commercialement, à l'exception des poudres, ces pigments interférentiels multicouche sont employés à une concentration massique n'excédant pas 5 %.

Il est connu par ailleurs par les fards à paupières de marque CHRYSALIDE de la société LANCÔME de conférer à la fois couvrance et intensité colorielle au moyen d'un maquillage faisant intervenir un premier geste consistant à déposer sur les matières kératiniques une couche de base de couleur noire contenant un pigment diffusant apportant de la couvrance et de déposer ensuite par un second geste sur cette couche de base une composition apportant de la couleur par le biais d'un pigment interférentiel multicouche. Sans la couche de base, la couche de recouvrement est pratiquement invisible car non couvrante et sans couleur.

Le recours à deux applications successives complique le maquillage et rend le conditionnement plus coûteux.

## Résumé

Composition avec un agent de coloration sensible à au moins un stimulus extérieur ou avec un pigment réfléchissant à reflet métallique ou avec un pigment diffractant.

Il existe un besoin pour bénéficier d'une composition capable d'apporter de la couvrance et de produire une couleur saturée, afin de pouvoir obtenir en un seul geste un maquillage à la fois couvrant et coloré.

Il existe également un besoin pour disposer d'une composition qui présente de nouveaux effets.

Par ailleurs, il est souhaitable de bénéficier de compositions cosmétiques destinées au maquillage des matières kératiniques qui présentent de nouveaux effets susceptibles d'attirer les consommateurs, sans que ces nouveaux effets ne se produisent au détriment de la qualité du maquillage obtenu.

L'invention vise notamment à répondre à tout ou partie des besoins identifiés ci-dessus.

L'invention a pour objet, selon l'un de ses aspects, une composition cosmétique comportant, dans un milieu cosmétiquement acceptable, au moins un pigment interférentiel multicouche et au moins l'un d'un agent de coloration sensible à au moins un stimulus extérieur, d'un pigment réfléchissant à reflet métallique et d'un pigment diffractant, la couvrance de la composition étant supérieure ou égale à 25, mieux 30, le pigment interférentiel multicouche étant en une teneur conférant à la composition un écart de couleur $\Delta E$ supérieur ou égal à 5 entre la couleur dans la masse et la couleur après application.

L'invention a pour objet, selon l'un de ses aspects, une composition cosmétique comportant, dans un milieu cosmétiquement acceptable, au moins un pigment interférentiel multicouche et au moins un agent de coloration sensible à au moins un stimulus extérieur (appelé par la suite Xchrome), la couvrance de la composition étant supérieure ou égale à 25, mieux 30, le pigment interférentiel multicouche étant en une teneur conférant à la composition un écart de couleur $\Delta E$ supérieur ou égal à 5 entre la couleur dans la masse et la couleur après application.

L'invention a également pour objet, selon l'un de ses aspects, une composition cosmétique comportant, dans

un milieu cosmétiquement acceptable, au moins un pigment interférentiel multicouche et au moins un pigment réfléchissant à reflet métallique, la couvrance de la composition étant supérieure ou égale à 25, mieux 30, le pigment interférentiel multicouche étant en une teneur conférant à la composition un écart de couleur ΔE supérieur ou égal à 5 entre la couleur dans la masse et la couleur après application.

L'invention a encore pour objet, selon l'un de ses aspects, une composition cosmétique comportant, dans un milieu cosmétiquement acceptable, au moins un pigment interférentiel multicouche et au moins un pigment diffractant, la couvrance de la composition étant supérieure ou égale à 25, mieux 30, le pigment interférentiel multicouche étant en une teneur conférant à la composition un écart de couleur ΔE supérieur ou égal à 5 entre la couleur dans la masse et la couleur après application.

La couleur après application est déterminée pour les besoins du calcul de ΔE après étalement de la composition sur une carte de contraste, comme pour la mesure de la couvrance.

L'agent de coloration sensible à au moins un stimulus extérieur peut être apte à prendre, dans un état, une couleur de longueur d'onde dominante éloignée de Δλ de moins de 30 nm de la longueur d'onde dominante de la composition dans la masse. Une telle caractéristique peut permettre par exemple d'avoir l'agent de coloration Xchrome et le pigment interférentiel multicouche sensiblement de la même couleur. En variante, l'agent de coloration Xchrome peut être incolore dans la composition en masse, ce qui peut être préférable lorsque celle-ci est blanche notamment.

L'agent de coloration sensible à au moins un stimulus extérieur peut être apte à prendre, dans un état, une couleur de longueur d'onde dominante éloignée de Δλ de moins de 30 nm de la longueur d'onde dominante de la composition après application sur des matières kératiniques. Cela peut permettre de renforcer la couleur de la composition après application.

L'agent de coloration sensible à au moins un stimulus extérieur peut être incolore dans un état, qui correspond par exemple à la présence du stimulus ou à l'absence de stimulus. Cet état peut se rencontrer dans la masse de la composition, avant l'application sur des matières kératiniques ou après l'application sur les matières kératiniques, en variante.

Selon les combinaisons retenues, l'invention offre la possibilité de créer de multiples effets.

L'agent de coloration sensible à au moins un stimulus extérieur peut être en solution dans le milieu ou être dispersé sous forme particulaire dans le milieu, selon sa nature.

L'agent de coloration sensible à au moins un stimulus extérieur est par exemple un agent photochrome, thermochrome, solvatochrome, piézochrome, tribochrome ou mécano luminescent.

L'invention peut ne comporter qu'un seul agent de coloration Xchrome ou en variante plusieurs agents de coloration Xchromes sensibles à des stimuli de différentes natures.

La composition peut être anhydre ou aqueuse.

Selon les compositions, l'écart ΔE peut être compris entre 5 et 30 par exemple, notamment supérieur à toute valeur entière comprise dans cet intervalle.

La couvrance peut être comprise entre 30 et 70, notamment supérieure à toute valeur entière comprise dans cet intervalle, par exemple supérieure ou égale à 40.

Dans les pigments interférentiels multicouche, la production de la couleur par le phénomène d'interférences est en compétition avec la production de la couleur par le phénomène d'absorption par la couche de surface du pigment.

Ainsi, lorsque la concentration en pigment augmente suffisamment, la couleur produite par le phénomène d'interférences diminue au profit de celle produite par absorption.

L'invention, en exploitant cette propriété, permet d'observer une variation de couleur de la composition à l'application, qui confère un aspect ludique à l'utilisation.

La variation de couleur à l'application peut être amplifiée ou nuancée par la présence de l'agent de coloration sensible à un stimulus extérieur, qui peut lui aussi changer de couleur à l'application, le cas échéant.

L'invention offre également de nouvelles possibilités en matière de commercialisation des compositions cosmétiques, en permettant de jouer au niveau du conditionnement sur la variation de couleur avant et après application.

Le ratio $m_1/m_2$ entre la teneur massique $m_1$ en pigment interférentiel multicouche et la teneur $m_2$ en agent de coloration Xchrome ou en pigment réfléchissant à reflet métallique ou en pigment diffractant va par exemple de 0,1 à 1,5.

La teneur massique en pigment interférentiel multicouche peut aller de 7 à 20 %, mieux 8 à 15 %, notamment dans le cas d'une composition non pulvérulente, par exemple liquide ou coulée.

Pour une composition pulvérulente, libre ou compactée, la teneur en pigment interférentiel multicouche va par exemple de 40 à 95 %, mieux 50 à 80 %.

La couleur de la composition dans la masse peut être blanche, lorsque la composition est non exposée au stimulus. Par « blanc » il faut comprendre achromatique au sens de la CIE.

La composition, en masse, peut avoir un indice de blancheur supérieur ou égal à 40.

La composition peut ne pas comporter d'autre agent de coloration que le ou les pigments interférentiels multicouche et :

- le ou les agents de coloration sensibles à un stimulus extérieur, ou
- le ou les pigments réfléchissants à reflet métallique, ou
- le ou les pigments diffractants.

Le pigment interférentiel multicouche peut comporter au moins quatre couches, par exemple.
Le pigment interférentiel multicouche peut comporter un substrat en une matière plus ou moins rugueuse, ce qui peut permettre de conférer plus de brillance à la composition.
Le substrat est par exemple choisi en mica naturel, relativement rugueux, en mica synthétique, en alumine, silice ou en verre ou métal pour une surface plus lisse.
La composition peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.
Dans des exemples, la composition comporte plus de 30 % en masse au total d'huile(s).
Dans des exemples, la composition comporte plus de 10 % en masse au total de cire(s).
Dans des exemples, la composition comporte plus de 10 % en masse au total de charge(s).
L'invention a encore pour objet, selon un autre de ses aspects, une composition cosmétique non pulvérulente, par exemple liquide (à 25 °C) ou sous forme de stick, comportant, dans un milieu cosmétiquement acceptable :

- entre 7 et 20 %, mieux 8 à 15 % en masse, au total, d'un ou plusieurs pigments interférentiels multicouche,
- entre 0,1 et 10 % en masse, au total, d'un ou plusieurs agents de coloration sensibles à au moins un stimulus extérieur ou d'un ou plusieurs pigments réfléchissants à reflet métallique ou d'un ou plusieurs pigments diffractants.

L'invention a encore pour objet, selon un autre de ses aspects, une composition pulvérulente, libre ou compactée, comportant, dans un milieu cosmétiquement acceptable :

- entre 40 et 95 %, mieux 50 et 80 %, encore mieux 55 et 65 % en masse, au total, d'un ou plusieurs pigments interférentiels multicouche,
- entre 0,1 et 60 %, mieux 1 et 50 % en masse, au total, d'un ou plusieurs agents de coloration sensibles à au moins un stimulus extérieur ou d'un ou plusieurs pigments réfléchissants à reflet métallique ou d'un ou plusieurs pigments diffractants.

Selon ces deux derniers aspects, la composition peut présenter l'une ou plusieurs des caractéristiques mentionnées plus haut, relatives par exemple à l'agent de coloration Xchrome ou au pigment réfléchissant à reflet métallique ou au pigment diffractant et à sa relation avec le pigment interférentiel multicouche.

<u>Composition blanche dans la masse</u>

L'invention a encore pour objet, selon l'un de ses aspects, indépendamment ou en combinaison avec ce qui précède, une composition cosmétique solide, notamment en stick, comportant, dans un milieu cosmétiquement acceptable, au moins un pigment interférentiel multicouche apte à conférer à la composition une couleur blanche dans la masse, et à provoquer après application une variation de couleur $\Delta E$ de la composition d'au moins 5.
Par « stick », encore appelé parfois « raisin » ou « bâton », on désigne une composition sous forme solide, généralement sous forme de bloc allongé, permettant un transfert sur les matières à maquiller par friction. Un stick peut être obtenu par exemple par moulage ou extrusion.
La couleur après application est déterminée pour les besoins du calcul de $\Delta E$ après étalement de la composition sur une carte de contraste, comme pour la mesure de la couvrance.
La composition, en masse, peut avoir un indice de blancheur supérieur ou égal à 40.
La composition peut être anhydre ou aqueuse.
Selon les compositions, l'écart $\Delta E$ peut être par exemple compris entre 5 et 30, notamment être supérieur à toute valeur entière comprise dans cet intervalle.
La couvrance de la composition peut être comprise entre 30 et 70, notamment être supérieure à toute valeur entière comprise dans cet intervalle, par exemple supérieure ou égale à 40.
La teneur massique en pigment interférentiel multicouche peut aller de 7 à 20 %, mieux 8 à 15 %, notamment dans le cas d'une composition non pulvérulente, par exemple liquide ou en stick.
Pour une composition pulvérulente, libre ou compactée, la teneur en pigment interférentiel multicouche va par exemple de 40 à 95 %, mieux 50 à 80 %.
La composition peut ne pas comporter d'autre agent de coloration que le ou les pigments interférentiels multicouche.

L'invention a encore pour objet, selon un autre de ses aspects, indépendamment ou en combinaison avec ce qui précède, une composition cosmétique comportant, dans un milieu cosmétiquement acceptable, au moins un pigment interférentiel multicouche apte à conférer à la composition une couleur blanche dans la masse, d'indice de blancheur supérieur ou égal à 40, la teneur massique total en pigment interférentiel multicouche allant de 7 à 20 % dans le cas d'une composition non pulvérulente et de 40 à 95 %, mieux 40 à 80%, dans le cas d'une composition pulvérulente.

L'invention a encore pour objet, selon un autre de ses aspects, indépendamment ou en combinaison avec ce qui précède, un ensemble de conditionnement comportant :

- une composition blanche dans la masse telle que définie ci-dessus,
- un moyen permettant de renseigner l'utilisateur sur la couleur de la composition après application sur des matières kératiniques (peau, lèvres, cheveux, ongles cils, sourcils). Il peut s'agir par exemple d'une impression d'une encre ou d'un vernis, d'une couche mince de composition ou d'un moulage ou surmoulage d'un matériau incorporant le ou les pigments interférentiels multicouche.

<u>Ensemble pour application bi-couche</u>

L'invention a encore pour objet, selon l'un de ses aspects, indépendamment ou en combinaison avec ce qui précède, un ensemble comportant :

- une première composition cosmétique à appliquer sur des matières kératiniques, par exemple l'une des compositions ci-dessus comportant l'une au moins d'un agent Xchrome, d'un pigment réfléchissant à reflet métallique ou d'un pigment diffractant, comportant dans un milieu cosmétiquement acceptable au moins un pigment interférentiel multicouche, la première composition présentant une couvrance supérieure ou égale à 25, mieux 30, la teneur en pigment interférentiel multicouche conférant à la composition un changement de couleur $\Delta E$ d'au moins 2, mieux 5 entre la couleur dans la masse et celle après application sur les matières kératiniques,
- une deuxième composition appelée composition de recouvrement ou *top coat,* à appliquer sur la première, ou
- une deuxième composition appelée composition de base ou *base coat,* à appliquer avant la première sur les matières kératiniques.

La deuxième composition est de préférence liquide.

La deuxième composition peut comporter une phase grasse, afin de conférer de la brillance au maquillage.

La deuxième composition peut être transparente, afin de ne pas affecter la saturation de la couleur produite par la première composition.

La deuxième composition peut être dépourvue de corps solides, afin de ne pas diffuser la lumière réfléchie par la première composition. En variante, la deuxième composition peut comporter au moins un pigment à effet, notamment un pigment réfléchissant métallique des pigments interférentiels, des composés à propriétés Xchromiques.

La première composition peut être anhydre ou aqueuse.

Selon les premières compositions, l'écart $\Delta E$ peut être supérieur à toute valeur entière comprise dans l'intervalle 5 à 30.

La couvrance de la première composition ou de la composition de base peut être supérieure à 30, notamment être supérieure à toute valeur entière comprise dans l'intervalle 30 à 70, par exemple supérieure ou égale à 40.

La teneur massique en pigment interférentiel multicouche dans <u>la première composition</u> peut aller de 7 à 15 %, mieux 8 à 15 %, notamment dans le cas d'une première composition non pulvérulente, par exemple liquide ou en stick.

Pour une première composition pulvérulente, la teneur en pigment interférentiel multicouche va par exemple de 40 à 95 %, mieux 40 à 80 %.

La couleur de la première composition dans la masse peut être blanche c'est-à-dire achromatique au sens de la CIE. La composition de base, en masse, peut avoir un indice de blancheur supérieur ou égal à 40.

La première composition peut ne pas comporter d'autre agent de coloration que le ou les pigments interférentiels multicouche.

La première composition peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.

L'ensemble peut comporter un moyen pour indiquer la couleur après application de la première composition ou après application des deux compositions de l'ensemble, sur les matières kératiniques (peau, lèvres, ongles, cils, sourcils, cheveux).

Il peut s'agir par exemple d'une impression d'une encre ou d'un vernis, d'une couche mince de composition ou d'un moulage ou surmoulage d'un matériau incorporant le ou les pigments interférentiels multicouche.

La deuxième composition de recouvrement comporte un milieu cosmétiquement acceptable.

La formulation de ce milieu est choisie de façon à permettre l'application sur la première composition, afin par exemple de conférer de la brillance et/ou améliorer la tenue et/ou nuancer un effet optique apporté par la première composition.

La deuxième composition de recouvrement peut comporter un corps gras liquide ou un agent filmogène.

La deuxième composition de recouvrement peut comporter un colorant et un ou plusieurs actifs et autres composés.

La deuxième composition de recouvrement peut présenter toute forme galénique compatible avec l'application sur la première composition.

Lorsque l'obtention de brillance est recherchée, la composition de recouvrement est de préférence liquide et transparente, comportant avantageusement une phase grasse.

La deuxième composition peut comporter au moins un pigment à effet. Ce pigment sera dans une teneur qui n'affecte pas l'observation de la couleur produite par la composition de base une fois appliquée sur les matières kératiniques.

Chaque composition peut être conditionnée dans tout réceptacle ou sur tout support prévu à cet effet. Les première et deuxième compositions peuvent être contenues, le cas échéant, dans deux compartiments d'un même dispositif de conditionnement et/ou dans un même emballage avant la première utilisation.

Chaque composition peut être appliquée en utilisant un applicateur, floqué ou non, par exemple une mousse, un embout, un pinceau, un feutre, une spatule, un fritté, une brosse, un peigne, un tissé ou non tissé.

L'application peut encore s'effectuer avec le doigt ou en déposant directement chaque composition sur le support à maquiller, par exemple par friction dans le cas d'un stick ou par pulvérisation ou projection à l'aide d'un dispositif piézoélectrique ou électrostatique ou par transfert d'une couche de composition préalablement déposée sur un support intermédiaire.

La première composition peut être conditionnée dans un dispositif de conditionnement qui laisse apparaître la couleur de la composition dans la masse.

Il peut s'agir d'un récipient ayant un corps au moins partiellement transparent et/ou d'un dispositif de conditionnement comportant un organe de bouchage au moins partiellement transparent.

La première composition peut être conditionnée dans un dispositif qui permet de voir à la fois la couleur de la composition dans la masse et la couleur de la composition après application sur des matières kératiniques.

La première composition peut encore être conditionnée dans un dispositif qui comporte des moyens représentatifs de la couleur révélée à l'application, par exemple un dépôt d'une couche de la première composition ou d'une encre ou vernis comportant les mêmes agents de coloration que la première composition.

Le cas échéant, la première composition ou la deuxième composition de recouvrement peut être conditionnée avec un aimant permettant de modifier l'orientation des particules de pigment interférentiel multicouche, lorsque ce pigment présente une susceptibilité magnétique non nulle.

<u>Stick multicolore</u>

L'invention a encore pour objet, selon l'un de ses aspects, indépendamment ou en combinaison avec ce qui précède, un produit solide, comportant au moins :

- un premier bloc d'une première composition cosmétique contenant, dans un milieu cosmétiquement acceptable, au moins un premier pigment interférentiel multicouche,
- un deuxième bloc d'une deuxième composition cosmétique, contenant dans un milieu cosmétiquement acceptable au moins un deuxième pigment interférentiel multicouche différent du premier,

l'une au moins des première et deuxième compositions étant apte à présenter une première couleur dans la masse et une deuxième couleur après application, les première et deuxième couleurs différant d'un écart $\Delta E$ supérieur ou égal à 2, mieux 5.

Chacune des première et deuxième compositions peut être apte à présenter une première couleur dans la masse et une deuxième couleur, après application sur des matières kératiniques, les première et deuxième couleurs de chaque composition différant d'un écart $\Delta E$ supérieur ou égal à 5.

Les premier et deuxième blocs peuvent s'étendre sur toute la longueur d'un stick (encore appelé parfois raisin ou bâtonnet) ou sur toute l'épaisseur du produit lorsque celui-ci est coulé ou compacté dans une coupelle.

Les premier et deuxième blocs peuvent être concentriques ou disposés autrement.

Selon les compositions, l'écart $\Delta E$ peut être compris par exemple entre 5 et 30.

La couvrance de l'une des compositions peut être supérieure à 25, par exemple comprise entre 30 et 70. La couvrance de chaque composition peut être supérieure ou égale à 25, mieux 30. De préférence, la couvrance de chaque composition est sensiblement la même. La couvrance du produit fondu puis étalé avec mélange des compositions des

différents blocs peut être supérieure ou égale à 25, par exemple comprise entre 30 et 70.

Selon cet aspect, l'invention offre un moyen de surprendre le consommateur en apportant la possibilité de révéler plusieurs couleurs à l'application, dans certains cas.

La teneur massique en pigment interférentiel multicouche dans l'une au moins des compositions peut aller de 7 à 20 %, mieux 8 à 15 %.

La couleur de l'une au moins des compositions dans la masse peut être blanche. Tous les blocs peuvent être blancs, c'est-à-dire achromatique au sens de la CIE, d'un indice de blancheur supérieur ou égal à 40 par exemple.

L'une au moins des compositions peut ne pas comporter d'autre agent de coloration que le ou les pigments interférentiels multicouche. Ce peut être le cas pour toutes les compositions du produit.

Les compositions du produit peuvent comporter deux pigments interférentiels multicouche respectifs ayant des couches constituées des mêmes matériaux.

Au moins une couche d'un pigment de l'une des compositions peut avoir une épaisseur différente d'une couche correspondante d'un pigment de l'autre composition, de façon à produire des couleurs différentes.

L'un au moins des compositions du stick multicolore peut comporter un agent Xchrome, un pigment diffractant et/ou un pigment à reflet métallique.

L'invention a encore pour objet, selon un autre de ses aspects, indépendamment ou en combinaison avec ce qui précède, un ensemble de conditionnement comportant :

- un produit solide tel que défini ci-dessus,
- un moyen permettant de renseigner l'utilisateur sur la couleur de l'une au moins des compositions du produit après application, voire d'une couleur formée par la superposition des compositions à l'application. Il peut s'agir par exemple d'une impression d'une encre ou d'un vernis, d'une couche mince de composition ou d'un moulage ou surmoulage d'un matériau incorporant le ou les pigments interférentiels multicouche.

Au sens de la présente invention, on entend englober par « produit solide » un produit ayant la propriété d'être dénué de la faculté d'écoulement sous l'action de son propre poids, dans des conditions normales de stockage.

Un produit solide peut être un produit dont la viscosité n'est pas mesurable.

Le produit solide peut, le cas échéant, présenter un aspect pâteux à température ambiante (25 °C).

L'un des blocs au moins pourra présenter un point de fusion ou une température de transition thermique (par exemple un point de ramollissement) supérieure à 25 °C, notamment pouvant varier de 25 à 85 °C, voire de 30 à 60 °C et en particulier de 30 à 45 °C.

La dureté de l'un au moins des blocs de composition pourra varier par exemple de 0,001 à 0,5MPa, notamment de 0,005 à 0,4MPa.

La dureté d'un bloc peut être déterminée par la mesure de la force en compression à 20 °C à l'aide du texturomètre vendu sous la dénomination TA-XT2i® par la société RHEO, équipé d'un cylindre en inox d'un diamètre de 2 mm se déplaçant à la vitesse de mesure de 0,1 mm/s, et pénétrant dans la composition de ce bloc à une profondeur de pénétration de 0,3 mm.

La valeur de la dureté est la force de compression maximale mesurée divisée par la surface du cylindre du texturomètre en contact avec la composition.

Dans le cas particulier des rouges à lèvres, la dureté peut également être mesurée par la méthode dite du fil à couper le beurre, qui consiste à couper un stick de rouge à lèvres de 8,1 mm de diamètre et à mesurer la dureté à 20 °C, au moyen d'un dynamomètre DFGHS 2 de la société Indelco-Chatillon se déplaçant à une vitesse de 100 mm/minute.

La dureté mesurée est exprimée comme la force de cisaillement (exprimée en grammeforce) nécessaire pour couper le stick dans ces conditions. Selon cette méthode, la dureté d'un produit solide selon l'invention va par exemple de 50 à 300 g, par exemple de 100 à 250 g et par exemple de 120 à 230 g.

**<u>Mesure de la couvrance</u>**

<u>Compositions liquides (à 25°C)</u>

Par « composition liquide », on entend une composition dont on peut mesurer la viscosité. Une composition liquide peut s'écouler sous l'effet de son propre poids.

La couvrance des compositions est mesurée à épaisseur finie de 50 $\mu$m, les compositions liquides étant par exemple des compositions à appliquer sur les lèvres, notamment des rouges à lèvres liquides, brillants à lèvres liquides et baumes à lèvres liquides, des vernis à ongles, des fards à paupières, des fonds de teint liquides, des mascaras et autres produits de maquillage liquides non destinés à être à appliqués sur les lèvres.

La composition est étalée sur des cartes de contraste noir mat et blanc mat, par exemple de marque LENETA Form WP1 pour la carte noir mat et Leneta 1A pour la carte blanc mat.

L'application peut s'effectuer avec un étaleur automatique.
Les mesures s'effectuent sur les compositions ainsi étalées.

Compositions solides (à 25°C)

Les compositions solides sont celles dont on ne peut mesurer la viscosité.
Il peut s'agir de compositions coulées en stick ou pulvérulentes, sous forme de poudres libres ou compactées.

a) Pour les compositions solides pulvérulentes, libres ou compactées, la composition est appliquée en utilisant les mêmes cartes de contraste que ci-dessus, recouvertes d'un ruban adhésif transparent, légèrement rugueux, par exemple de marque BLENDERM® de la société 3M et de référence 15025, collé par la face adhésive sur les cartes de contraste.

La composition est déposée sur le ruban adhésif de manière à obtenir un dépôt homogène de 0,5 $mg/cm^2 \pm$ 0,02 $mg/cm^2$.
Il est possible d'utiliser pour effectuer le dépôt une éponge chargée avec la composition et montée sur un appareil de délitage qui fait effectuer des mouvements prédéfinis à l'éponge. L'éponge est par exemple une éponge à usage unique de type « LANCÔME - Photogenic », utilisée du coté rose.

b) Les compositions en stick sont fondues, par exemple à 90°C, puis étalées avec une épaisseur de 50 μm à l'état liquide sur des cartes de contraste noir mat et blanc mat, par exemple de mêmes références que ci-dessus, non recouvertes de BLENDERM®. Le barreau d'étalement est maintenu à la même température que la composition, de façon à éviter un choc thermique.

Mesures et calculs

Des spectres de réflectance sont acquis à l'aide d'un spectrocolorimètre MINOLTA 3700-d (géométrie de mesure diffuse/8° et observation D65/10°, mode composante spéculaire exclue, petite ouverture (CREISS)) sur les fonds noir et blanc, les cartes de contraste étant éventuellement recouvertes de BLENDERM® comme indiqué ci-dessus.
Les spectres sont exprimés en coordonnées colorimétriques dans l'espace CIELab76 au sens de la Commission Internationale de l'Eclairage selon la recommandation 15:2004.
Le contraste ratio, ou couvrance, est calculé en faisant la moyenne arithmétique de Y sur fond noir, divisée par la valeur moyenne de Y sur fond blanc, multiplié par 100.

**Mesure de la couleur de la composition dans la masse**

La couleur dans la masse est mesurée après avoir rempli un contenant présentant une profondeur suffisamment importante pour pouvoir considérer une épaisseur de produit infinie au vu de la mesure, par exemple une profondeur de 3 mm ou plus.
Les coordonnées L*, a* et b* sont mesurées avec un spectrocolorimètre, par exemple de marque MINOLTA CM-2002 (D65/10°, mode composante spéculaire exclue).

**Mesure de la couleur après application**

La couleur est mesurée sur le fond sombre de la carte de contraste, la composition étant étalée comme pour la mesure de la couvrance ainsi que détaillé ci-dessus mais avec une épaisseur de 150 μm au lieu de 50 μm pour les compositions liquides et les compositions solides non pulvérulentes, notamment en stick.
L'écart ΔE est calculé comme suit :

$$\Delta E = \left[ \left( a*_{dans\,la\,masse} - a*_{après\,application} \right)^2 + \left( b*_{dans\,la\,masse} - b*_{après\,application} \right)^2 + \left( L*_{dans\,la\,masse} - L*_{après\,application} \right)^2 \right]^{1/2}$$

Lorsque l'écart $\Delta E$ varie en fonction de l'angle d'observation en raison de la présence d'un agent de coloration goniochromatique, c'est l'écart maximal qui est retenu.

**Mesure de l'indice de blancheur**

L'indice de blancheur est calculé d'après les mesures de couleur à épaisseur infinie et selon la norme ASTM E313-O5.

**Pigment interférentiel multicouche**

L'expression « pigment interférentiel multicouche » désigne un pigment capable de produire une couleur par un phénomène d'interférences entre les rayonnements lumineux réfléchis par une pluralité de couches superposées d'indices de réfraction différents, notamment une succession de couches de haut et de bas indices de réfraction. Le pigment peut comporter un substrat, par exemple de mica, recouvert d'une seule couche, par exemple de $TiO_2$.

Tous les pigments interférentiels multicouche peuvent être envisagés.

La couleur produite par le pigment interférentiel multicouche peut être quelconque, étant par exemple de longueur d'onde dominante comprise entre 580 et 650 nm ou non.

La composition peut comporter un seul pigment interférentiel multicouche ou plusieurs pigments interférentiels multicouche ayant des longueurs d'onde dominantes différentes.

Le pigment interférentiel multicouche peut comporter un substrat (encore appelé coeur ou noyau) recouvert sur au moins une face d'une ou plusieurs couches en des matériaux et épaisseurs choisis de telle sorte qu'une couleur soit produite par interférences.

Des couches du pigment interférentiel peuvent entourer ou non le substrat, lequel peut présenter une forme aplatie ou non.

Le substrat peut comporter du mica naturel, du mica synthétique, du verre, de l'alumine, de la silice, ou encore un quelconque métal, alliage ou oxyde métallique. La nature du substrat pourra être choisie en fonction de la brillance souhaitée. Par exemple, pour un rendu très brillant, un substrat en verre ou en métal pourra être préféré.

Le pigment interférentiel peut comporter plus de quatre couches d'indices de réfraction différents.

La taille des particules du pigment interférentiel multicouche, donnée par la granulométrie moyenne à la moitié de la population, encore appelée $D_{50}$, va par exemple de 1 $\mu$m à 2000$\mu$m, mieux de 5 $\mu$m à 2000 $\mu$m.

La proportion de pigment interférentiel multicouche est par exemple supérieure à 7 % et va par exemple de 7 à 20 % pour une composition non pulvérulente, liquide ou coulée, par exemple en stick et de 40 à 95 % pour une composition pulvérulente, libre ou compactée.

La couvrance de la composition peut être due essentiellement à la teneur en pigment interférentiel multicouche. En variante, au moins un pigment diffusant et/ou des charges peuvent conférer de la couvrance.

Les nacres sont des exemples de pigments interférentiels multicouche pouvant convenir.

Nacres

Par « nacre », il faut comprendre des particules colorées de toute forme, irisées ou non, notamment produites par certains mollusques dans leur coquille ou bien synthétisées et qui présentent un effet de couleur par interférence optique.

Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment ainsi que les pigments nacrés à base d'oxychlorure de bismuth.

Il peut également s'agir de particules de mica à la surface desquelles sont superposées au moins deux couches successives d'oxydes métalliques et/ou de matières colorantes organiques.

Les nacres peuvent présenter une couleur ou un reflet jaune, rose, rouge, bronze, orangé, brun, or et/ou cuivré.

A titre illustratif de nacres pouvant être introduites en tant que pigment interférentiel multicouche, on peut citer les nacres de couleur or notamment commercialisées par la société ENGELHARD sous le nom de Brillant gold 20 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à

reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Des pigments interférentiels multicouche présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO 25 TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMA-LITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

Le pigment interférentiel peut avantageusement être choisi parmi ceux conférant une couleur blanche dans la masse à la composition, à savoir les nacres notamment commercialisées par la société ENGELHARD sous le nom de SPARKLE 110P (Timica), Flamenco blue (Flamenco), Flamenco green (Flamenco), Flamenco red (Flamenco), Flamenco violet (Flamenco), Flamenco orange (Flamenco),Silkbalnc 110W (Timica), Extra large sparkle (Timica), Flamenco sparkle Gold (Flamenco), Flamenco sparkle green (Flamenco), Flamenco sparkle orange (Flamenco), Flamenco sparkle blue (Flamenco), Flamenco sparkle violet (Flamenco), Flamenco sparkle red (Flamenco), Flamenco summit gold (Flamenco) ; les nacres commercialisées par la société MERCK sous la dénomination Silk blue Timiron (Mica/$TiO_2$/$SnO_2$), Silk green Timiron (Mica/$TiO_2$/$SnO_2$ ), Silk red Timiron (Mica/$TiO_2$/$SnO_2$), Super red Timiron (Mica/$TiO_2$), Super green Timiron (Mica/$TiO_2$), Super blue Timiron (Mica/$TiO_2$), Artic Silver Timiron, Splendid copper Timiron (Mica/$TiO_2$/$SiO_2$), Splendid Violet Timiron (Mica/$TiO_2$/$SiO_2$) ; les nacres notamment commercialisées par la société ECKART sous la dénomination Prestige Silver (Prestige), Prestige Silver Star (Prestige), Prestige Gold (Prestige), Prestige soft gold (Prestige), Prestige silk green (Prestige), Prestige silk lilac (Prestige), Prestige silk blue (Prestige), Prestige silk red (Prestige).

Le pigment interférentiel peut être dépourvu de couche d'un matériau coloré, notamment choisi parmi FeOOH, $Fe_2O_3$, $Cr_2O_3$, $TiO_{2-x}$, $TiO_xN_y$, $CrPO_4$, KFe[Fe(CN)$_6$], $Fe_3O_4$, TiO, TiN, $FeTiO_3$, C, Ag, Au, Fe, Mo, Cr, W.

Le pigment interférentiel peut par exemple ne comporter sur le substrat qu'une ou plusieurs couches des matériaux choisis parmi $TiO_2$ (rutile ou anatase), $ZrO_2$, $SnO_2$, $SiO_2$.

Le pigment interférentiel multicouche peut encore être choisi parmi les particules réfléchissantes interférentielles.

Particules réfléchissantes interférentielles

Ces particules peuvent être choisies parmi les particules à substrat synthétique enrobé au moins partiellement d'au moins une couche d'au moins un oxyde métallique, choisi par exemple parmi les oxydes de titane, notamment $TiO_2$, de fer notamment $Fe_2O_3$, d'étain, de chrome, le sulfate de baryum et les matériaux suivants : $MgF_2$, $CrF_3$, ZnS, ZnSe, $SiO_2$, $Al_2O_3$, MgO, $Y_2O_3$, $SeO_3$, SiO, $HfO_2$, $ZrO_2$, $CeO_2$, $Nb_2O_5$, $Ta_2O_5$, $MoS_2$ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD.

Le pigment interférentiel multicouche peut encore être un pigment goniochromatique.

Pigment goniochromatique

Par « pigment goniochromatique », on désigne au sens de la présente invention un pigment permettant d'obtenir, lorsque la composition est étalée sur un support, un trajet de couleur dans le plan a*b* de l'espace colorimétrique CIE 1976 qui correspond à une variation Dh° de l'angle de teinte h° d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition a été étalée à l'état fluide avec une épaisseur de 300 μm au moyen d'un étaleur automatique sur une carte de contraste de marque ERICHSEN et de référence Typ 24/5, la mesure étant effectuée sur le fond noir de la carte.

Le pigment goniochromatique peut être choisi par exemple parmi les structures multicouches interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

La structure multicouche peut présenter ou non, par rapport à une couche centrale, une symétrie au niveau de la nature chimique des couches empilées.

Selon l'épaisseur et la nature des différentes couches, on obtient différents effets.

Des exemples de structures multicouche interférentielles symétriques sont par exemple les structures suivantes : $Fe_2O_3/SiO_2/Fe_2O_3/SiO_2/Fe_2O_3$, un pigment ayant cette structure étant commercialisé sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$ ; $TiO_2/SiO_2/TiO_2$ et $TiO_2/Al_2O_3/TiO_2$, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE® HC par la société WACKER.

Comme pigment goniochromatique, on peut encore utiliser certaines nacres, des pigments à effets sur substrat synthétique, notamment substrat type alumine, silice, borosilicate, oxyde de fer, aluminium, ou des paillettes interférentielles issues d'un film de polytéréphthalate.

Le matériau peut en outre comporter des fibres goniochromatiques dispersées. De telles fibres pourront présenter une longueur inférieure à 80 μm par exemple.

## Agent de coloration Xchromes

Il peut s'agir d'agents de coloration photochromes.

### Agents de coloration photochromes

D'une manière générale, un agent de coloration photochrome est un agent colorant ayant la propriété de changer de teinte lorsqu'il est éclairé ou non par une lumière ultraviolette et de rétablir sa couleur initiale lorsqu'il n'est plus éclairé ou non par une lumière ou encore de passer d'un état non coloré à un état coloré et inversement. En d'autres termes, un tel agent présente des teintes différentes selon qu'il est éclairé par de la lumière contenant une certaine quantité de rayonnement UV.

L'agent de coloration photochrome peut prendre en présence d'un faible éclairage un état sensiblement non coloré.

En présence d'un fort éclairage, l'agent de coloration photochrome peut prendre un état coloré, par exemple une teinte foncée ou une couleur ayant sensiblement la même longueur d'onde dominante que le pigment interférentiel multicouche, ce qui peut permettre de faire paraître le pigment interférentiel multicouche moins brillant qu'en présence d'un éclairage faible. Cet effet peut surprendre l'observateur et rendre le maquillage particulièrement attractif.

L'agent de coloration photochrome peut présenter un écart $\Delta E$ au moins égal à 5. $\Delta E$ désigne l'écart de teinte observé au niveau de la matière photochromique entre son état excité, c'est-à-dire en présence d'irradiation UV et son état non excité, c'est-à-dire en absence d'irradiation UV.

On pourra utilement se référer aux exemples d'agents photochromes décrits dans US 2004/0228818, dont le contenu est incorporé par référence, notamment ceux présentant un $\Delta E$ supérieur à 5, conformément au test présenté dans ce document.

Comme exemples d'agents de coloration photochromes, figurent les dérivés naphtopyrane de type 2H-naphto-[2,1-b]-pyrane de formule (I) ou 3H-naphto-[2,1-b]-pyrane, de formule (II) :

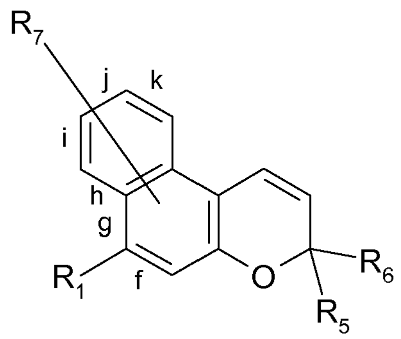

(I)

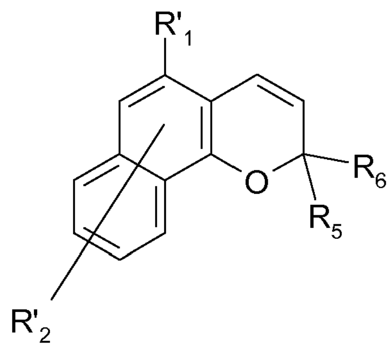

(II)

dans lesquels :

$R_1$ représente :

- (i) un atome d'hydrogène;
- (ii) un groupement hydrocarboné ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné;
- (iii) un cycle hydrocarboné formé avec l'une des liaisons "f" ou "gh" et le radical $R_7$, ou
- (iv) un groupement choisi parmi $-COOR_4$, $-C(O)NR_2R_3$, $-NR_2R_3$, $-OR_4$ et - $SR_4$, dans lequel :
- $R_2$ et $R_3$ soit représentent, indépendamment l'un de l'autre, un groupement hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,

soit pris ensemble avec l'atome d'azote auquel ils sont reliés, forment un hétérocycle hydrocarboné, saturé ou insaturé, comportant 3 à 10 atomes de carbone et éventuellement 1 à 5 autres hétéroatomes choisis parmi N, O, S, Si et P, ledit cycle étant éventuellement substitué par au moins un radical hydrocarboné ayant 1 à 20 atomes de carbone, linéaire, ramifié ou cyclique, saturé ou insaturé, comportant éventuellement 1 à 5 hétéroatomes choisis parmi N, O, S, Si et P;

$R_4$ représente un groupement hydrocarboné ayant 1 à 20 atomes de carbone, linéaire, ramifié ou cyclique, saturé ou insaturé, éventuellement halogéné ou perhalogéné, et/ou éventuellement comportant 1 à 5 hétéroatomes choisis parmi N, O, S, Si et P;

$R_5$ et $R_6$ représentent, indépendamment l'un de l'autre, un groupement choisi parmi :

- (i) les groupements aminoaryles cycliques saturés de formule (IIA) ou (IIB) :

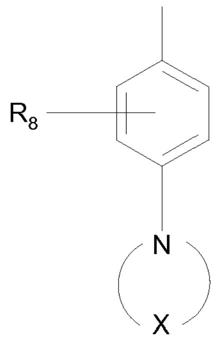

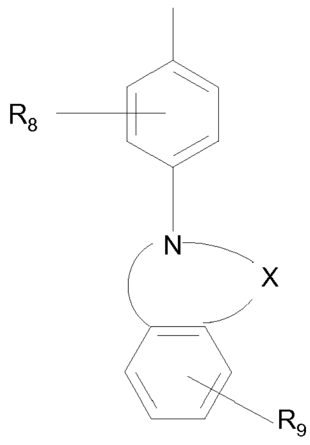

(IIA) (IIB)

dans lesquelles le cycle comportant N et X est un cycle saturé qui comprend au total 3 à 30 atomes inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P;

- (ii) les groupements indolinoaryles de formule (III) :

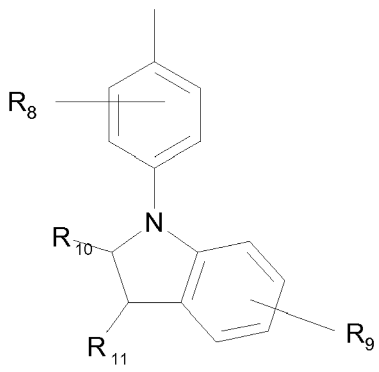

(III)

dans laquelle $R_{10}$ et $R_{11}$ représentent, indépendamment l'un de l'autre, un groupement choisi parmi (i) les groupements hydrocarbonés ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné ; (ii) les atomes d'halogène ; (iii) les groupements -CN (nitrile), -COOH (carboxylate), $-NO_2$ (nitro); (iv) un atome d'hydrogène; (v) un groupement choisi parmi - $C(O)NR_2R_3$, $-NR_2R_3$, $-OR_4$ ou $-SR_4$ avec $R_2$, $R_3$ et $R_4$ ayant les significations données ci-dessus; (vi) les radicaux $R_{10}$ et $R_{11}$ pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20, atomes de carbone, linéaire, ramifié ou cyclique, saturé ou insaturé, éventuellement comportant 1 à 5 hétéroatomes choisis parmi N, O, S, Si et P,

- (iii) les groupements de formule (IV) :

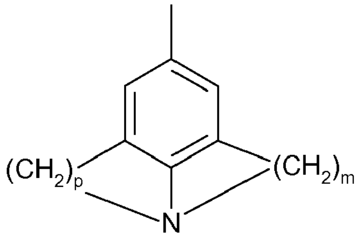

(IV)

dans laquelle m et p sont, indépendamment l'un de l'autre, des entiers allant de 2à5;

- (iv) les groupements aminoaryles cycliques insaturés de formules (VA), (VB) ou (VC) :

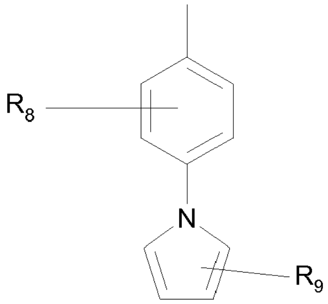

(VA)

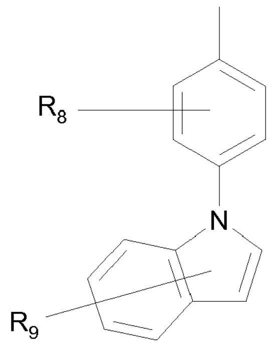

(VB)

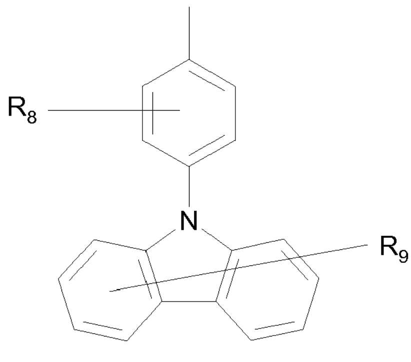

(VC)

dans lesquelles $R_8$ et $R_9$, représentent, indépendamment l'un de l'autre, un groupement choisi parmi (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ; (ii) les atomes d'halogène ; (iii) les groupements -CN (nitrile), -COOH (carboxylate), -NO2 (nitro); (iv) un atome d'hydrogène; (v) un groupement choisi parmi - $C(O)NR_2R_3$, $-NR_2R_3$, $-OR_4$ ou $-SR_4$ avec $R_2$, $R_3$ et $R_4$ ayant les significations données ci-dessus;

- (v) un groupement hydrocarboné ayant 1 à 30 atomes de carbone, linéaire, ramifié ou cyclique, saturé ou

insaturé, éventuellement comportant 1 à 5 hétéroatomes choisis parmi N, O, S, Si et P; et notamment un groupement choisi parmi $CONR_2R_3$, - $C_6H4-NR_2R_3$ et $-C_6H_4-OR_4$ avec $R_2$, $R_3$ et $R_4$ ayant les significations données ci-dessus;

$R_7$ représente un groupement choisi parmi :

- (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
- (ii) les atomes d'halogène ;
- (iii) les groupements -CN (nitrile), -COOH (carboxylate), -N02 (nitro); - N=N- (azo); =NH (imino); $-CONH_2$ (amide);
- (iv) un atome d'hydrogène;
- (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;
- (vi) le radical $R_7$ pouvant en outre former, avec l'une des liaisons "i", "j", "k", ou "g,h" prises avec le radical $R_1$, ou "f' prise avec le radical R1, un cycle hydrocarboné saturé ayant au total 3 à 8 atomes de carbone, éventuellement comportant 1 à 5 hétéroatomes choisis parmi N, O, S, Si et P;

R'1 représente un groupement choisi parmi :

- (i) un atome d'hydrogène;
- (ii) un groupement hydrocarboné ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
- (iii) un groupement choisi parmi $-C(O)NR_2R_3$, $-NR_2R_3$, $-OR_4$ et $-SR_4$, avec $R_2$, $R_3$ et $R_4$ ayant les significations données ci-dessus;

$R'_2$ représente un groupement choisi parmi :

- (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
- (ii) les atomes d'halogène ;
- (iii) les groupements -CN (nitrile), -COOH (carboxylate), $-NO_2$ (nitro); - N=N- (azo); =NH (imino); $-CONH_2$ (amide);
- (iv) un atome d'hydrogène;
- (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus.

Comme exemples d'agents photochromes, on peut encore citer le diaryléthène, de formule

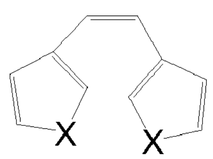

et ses dérivés,
le dihydroazulène/vinylhepta fulvène, de formule

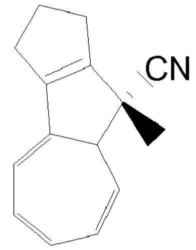

et ses dérivés,
le spyronaphtoxazine, de formule

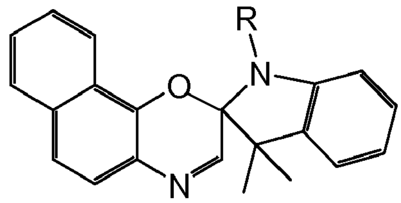

et ses dérivés.

L'agent photochrome peut être un composé organique ou inorganique. Lorsque l'agent photochrome est un composé organique, le changement de couleur peut généralement être plus rapide et intense.

A titre d'exemples d'agents photochromes, on peut citer le Photosol® de la société PPG, qui change de manière réversible de couleur lorsqu'activé par un rayonnement UV de longueur d'onde comprise entre 300 et 360 nm, le Reversacol® de la société J. ROBINSON et le Photogenica® de la société CATALYST & CHEMICALS.

Agents thermochromes

Un agent thermochrome est un pigment ou colorant qui peut changer de couleur en fonction de la température.

L'agent thermochrome présente par exemple une couleur qui est perdue lorsque la température dépasse une certaine valeur, par exemple 15 °C environ ou 30 °C environ, selon la nature de l'agent thermochrome.

L'agent thermochrome peut comporter des capsules d'un polymère contenant un solvant, ce solvant permettant, selon son étant fondu ou non, à des composés d'entrer en contact et de modifier des propriétés d'absorption du rayonnement lumineux.

Le changement de couleur peut être réversible.

On peut par exemple utiliser l'agent thermochrome commercialisé sous la référence Kromafast® Yellow5GX 02 par la société KROMACHEM LTD, ou encore Chromazone® en poudre ou dispersion, Thermobatch® ou Thermostar®, de la société CHROMAZONE.

Agents piézochromes et tribochromes

Un agent piézochrome est susceptible de changer de couleur en présence d'une force mécanique.

Comme exemples d'agents piézochromes, on peut citer le diphenylflavylene.

Un agent tribochrome est susceptible de changer de couleur en présence d'une force mécanique, de manière plus durable que dans le cas des agents piézochromes.

On pourra se référer à la publication WO 94/26729, dont le contenu est incorporé par référence.

Agents mécanoluminescents

Ces agents sont capables d'émettre une lumière lorsqu'ils reçoivent une sollicitation mécanique telle qu'une compression, un cisaillement ou un frottement.

L'agent mécanoluminescent est de préférence sous forme de particule insoluble dans le milieu cosmétique. La taille moyenne des particules est par exemple entre 0,01 et 50 µm, de préférence entre 0,1 et 10 µm.

Comme matériaux mécano luminescents, on peut citer :

a) les complexes et chélates de lanthanides tels que ceux décrits dans les publications US 6 071 632, US 2002/0015965 et WO 09/016429 dont les contenus sont incorporés par référence. Les terres rares sont de préférence choisies parmi l'europium, le terbium, le samarium et le dysprosium. Dans ces matériaux, des dicétones sont utilisées comme ligand des sels de lanthanide trivalent.

b) Les aluminates, silicates et aluminosilicates dopés avec des ions de terres rares tels que décrits dans les publications US 6 280 655, EP 1 318 184, JP 2002/194349, JP 2004/59746, dont les contenus sont incorporés par référence, notamment (Sr,Mg,Ba,Zn,Ca) $Al_2O_4$,(SrLa,SrY)$Al_3O_7$($Sr_2$,SrMg,SrCa,SrBa)$Al_6O_{11}$, $Sr_2$(Mg,Al)(Al,Si) $SiO_7$, Sr(Zn,Mn,Fe,Mg)$Si_2O_6$. Les éléments indiqués entre parenthèses sont partiellement ou entièrement interchangeables. Des ions de terres rares tels que le cérium, l'europium, le samarium, le néodymium, le gadolinium, le disprosium et le terbium peuvent être utilisés, seuls ou en mélange. L'europium et le disprosium sont préférés.

c) Le sulfure de zinc, le sulfure de manganèse, le sulfure de cuivre, le sulfure de cadmium ou l'oxyde de zinc, éventuellement dopés avec des ions de métaux de transition ou des ions de terres rares comme décrit dans les publications US 6 117 574 et JP 2004/43656 dont les contenus sont incorporés par référence. Les ions de métaux de transition sont de préférence le cuivre ou le manganèse. Les ions de terres rares sont de préférence l'europium ou le cérium. Parmi ces matériaux, ZnS : Mn est préféré.

Les matériaux listés sous b) et c) peuvent être synthétisés par une réaction en phase solide impliquant un mélange à sec suivi d'un traitement thermique et de frittage à haute température ou par un process sol-gel suivi d'un séchage, chauffage et frittage. La température de frittage est par exemple supérieure à 1 000 °C.

Les matériaux listés sous b) sont préférés. Parmi ceux-ci, $SrAl_2O_4$ et $SrMgAl_{10}O_{17}$ dopés avec des métaux rares sont préférés.

Des pigments mécano luminescents SrA12O4 dopés avec des ions de métaux rares sont vendus sous la référence TAIKO-M1-1 par la société TAIKO Refractories Co., Ltd. Les particules de ce pigment ont un diamètre compris entre 5 et 10 μm et une luminescence verte sous une faible contrainte mécanique.

Agents solvatochromes

Un agent solvatochrome est susceptible de changer de couleur en présence de solvants. Le colorant DC Red27 en est un exemple, ce composé présentant dans une formulation anhydre une absence de couleur et l'adjonction d'eau révélant une couleur rose.

**Pigment réfléchissant à réflexion métallique**

Le pigment réfléchissant à réflexion métallique n'est pas un pigment interférentiel multicouche au sens de l'invention.

La taille des particules réfléchissantes à reflet métallique du pigment réfléchissant à réflexion métallique peut aller par exemple de 10 μm à 500 μm, étant de préférence comprise entre 10 et 150 μm. La taille peut être supérieure ou égale à 40 μm afin de pouvoir être perçue visuellement.

Les particules réfléchissantes à reflet métallique peuvent présenter une forme plaquettaire ou globulaire, notamment sphérique, allongée ou non, avec le cas échéant un facteur de forme important. Une forme plaquettaire peut rendre la réflexion plus directionnelle. Au contraire, une forme sensiblement sphérique peut apporter une réflexion plus diffuse.

Les particules réfléchissantes à reflet métallique comportent par exemple au moins une couche conductrice de l'électricité en surface, formée d'au moins un métal ou oxyde métallique, éventuellement dopé.

Les particules réfléchissantes à reflet métallique peuvent présenter en surface au moins une couche protectrice suffisamment transparente pour conserver à la particule l'effet de réflexion métallique recherché.

Les particules réfléchissantes à reflet métallique, quelle que soit leur forme, peuvent présenter une structure multicouche ou non et, dans le cas d'une structure multicouche, comporter par exemple au moins une couche d'un métal ou alliage, éventuellement dopé. Cette couche peut être d'épaisseur uniforme.

Lorsque les particules réfléchissantes ne présentent pas de structure multicouche, elles peuvent être composées par exemple d'au moins un métal ou alliage métallique, éventuellement dopé.

Lorsque les particules réfléchissantes à reflet métallique présentent une structure multicouche, celles-ci peuvent comporter un substrat naturel ou synthétique, notamment un substrat synthétique au moins partiellement enrobé par au moins une couche d'au moins un métal, un alliage ou un oxyde métallique.

Cette couche est par exemple une couche extérieure de la structure. Le substrat peut être monomatière, multimatériau, organique et/ou inorganique. Plus particulièrement, le substrat peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

A titre d'exemple de particules réfléchissantes à reflet métallique comportant un substrat minéral enrobé d'une couche de métal, on peut citer les particules comportant un substrat de borosilicate enrobé d'argent. Des particules à substrat de verre revêtu d'argent, en forme de plaquettes, sont vendues sous la dénomination de MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Comme exemples de particules réfléchissantes à reflet métallique présentant en surface un composé métallique ou incluant au moins un composé métallique, éventuellement enrobé, on peut citer les particules proposées sous les dénominations METASHINE® ME 2040 PS, METASHINE® MC5090 PS ou METASHINE® MC280GP (2523) par la société NIPPON SHEET GLASS, SPHERICAL SILVER POWDER® DC 100, SILVER FLAKE® JV6 ou GOLD POWDER® A1570 par la société ENGELHARD, STARLIGHT REFLECTIONS FXM® par la société ENERGY STRATEGY ASSOCIATES INC. BRIGHT SILVER® 1 E 0.008X0.008 par la société MEADOWBROOK INVENTIONS, ULTRAMIN® (ALUMINIUM POUDRE FINE LIVING), et COSMETIC METALLIC POWDER VISIONAIRE BRIGHT SILVER SEA®, COSMETIC METALLIC POWDER VISIONAIRE NATURAL GOLD® (60314) ou COSMETIC METALLIC POWDER VISIONAIRE HONEY® (60316) par la société ECKART.

Les particules réfléchissantes à reflet métallique peuvent réfléchir le spectre visible de manière sensiblement uniforme, comme c'est le cas par exemple de particules revêtues d'un métal tel que l'argent ou l'aluminium, ou non, ce qui peut alors conduire par exemple à un reflet métallique ayant un ton non neutre, jaune, rose, rouge, bronze, orangé, brun, or et/ou cuivré, selon la nature par exemple du composé métallique en surface.

Les particules réfléchissantes à reflet métallique peuvent être présentes dans la composition à une teneur allant de 0,1 % à 60 % par rapport au poids total de la première composition, notamment de 1 % à 30 % en poids, par exemple de 3 % à 10 % en poids.

La teneur en particules réfléchissantes à reflet métallique sera suffisamment faible pour ne pas entraver la production de couleur par le pigment interférentiel multicouche.

Le pigment réfléchissant à réflexion métallique peut participer à l'obtention de la couvrance recherchée.

**Pigment diffractant**

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière.

Un tel pigment est encore parfois appelé pigment holographique ou à effet arc en ciel.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel. On pourra utilement se reporter concernant la structure des pigments diffractants à l'article « Pigments Exhibiting Diffractive Effects » d'Alberto Argoitia and Matt Witzman, 2002, Society of Vacuum coaters, 45th Annual Technical Conference, Proceedings 2002, dont le contenu est incorporé par référence.

Le pigment diffractant peut être réalisé avec des motifs ayant différents profils, notamment triangulaires, symétriques ou non, en créneaux, de largeur constante ou non, sinusoïdaux, en escalier.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette. Une même particule de pigment peut comporter deux réseaux de diffraction croisés, perpendiculaires ou non, de même linéature ou non.

Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : $MgF_2$, $SiO_2$, $Al_2O_3$, $AlF_3$, $CeF_3$, $LaF_3$, $NdF_3$, $SmF_2$, $BaF_2$, $CaF_2$, LiF et leurs associations.

Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs matériaux, associations ou alliages. Un tel matériau réfléchissant peut, seul, constituer le pigment diffractant qui sera alors monocouche.

En variante, le pigment diffractant peut comporter une structure multicouche comportant un substrat (encore appelé coeur ou noyau) d'un matériau diélectrique recouvert d'une couche réfléchissante au moins d'un côté, voire encapsulant complètement le substrat.

Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : $MgF_2$, SiO, $SiO_2$, $Al_2O_3$, $TiO_2$, WO, AIN, BN, $B_4C$, WC, TiC, TiN, $N_4Si_3$, ZnS, des particules de verre, des carbones de type diamant et leurs associations. En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de $SiO_2$, de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées. Le pigment diffractant utilisé peut notamment être choisi parmi ceux décrits dans la demande de brevet américain US 2003/0031870 publiée le 13 février 2003. Un pigment diffractant peut comporter par exemple la structure suivante : $MgF_2/Al/MgF_2$, un pigment diffractant ayant cette structure étant commercialisé sous la dénomination SPECTRAFLAIR 1400 Pigment Silver par la société FLEX PRODUCTS, 25 ou SPECTRAFLAIR 1400 Pigment Silver FG. La proportion en poids du $MgF_2$ peut être comprise entre 80 et 95 % du poids total du pigment. D'autres pigments diffractants sont commercialisés sous les dénominations METALURE® PRISMATIC par la société ECKART®.

D'autres structures possibles sont Fe/Al/Fe ou Al/Fe/Al.

La dimension du pigment diffractant peut être comprise par exemple entre 5 et 200 $\mu$m, mieux entre 5 et 100 $\mu$m, par exemple entre 5 et 30 $\mu$m. L'épaisseur des particules de pigment diffractant peut être inférieure ou égale à 3 $\mu$m, mieux 2 $\mu$m, par exemple de l'ordre de 1 $\mu$m.

## Milieu cosmétiquement acceptable

Par « milieu cosmétiquement acceptable », on désigne un milieu non toxique susceptible d'être appliqué sur les matières kératiniques d'êtres humains.

Le milieu cosmétiquement acceptable sera adapté à la nature du support sur lequel doit être appliqué la composition ainsi qu'à la forme sous laquelle la composition est destinée à être conditionnée.

La composition selon l'invention peut comprendre un milieu aqueux et/ou une phase grasse, être anhydre ou non.

### Phase aqueuse ou grasse

La composition peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols.

La phase hydrophile peut, en outre, contenir des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0 % à 90 %, notamment 0,1 % à 90 % en poids, par rapport au poids total de la composition, et de préférence de 0 % à 60 % en poids, notamment 0,1 % à 60 % en poids.

La composition peut également comprendre une phase grasse, notamment constituée de corps gras liquides à 25 °C et éventuellement de corps gras solides à température ambiante tels que les cires, les corps gras pâteux, les gommes et leurs mélanges.

Comme corps gras liquides à température ambiante, appelés souvent huiles, utilisables dans l'invention, on peut citer entre autres les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et, notamment les isoparaffines en $C_8$-$C_{16}$ telles que l'isododécane, l'isodécane, l'isohexadécane, la vaseline, les polydécènes, le polyisobutène hydrogéné tel que le Parléam®, le squalane; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ;

des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 % par rapport au poids total de la composition.

La composition selon l'invention peut également comprendre un ou plusieurs solvants organiques, physiologiquement acceptables. Ce ou ces solvants, qui peuvent être lipophiles, peuvent être présents en une teneur allant de 0 à 90 %, mieux de 0 à 60% en poids, par rapport au poids total de la composition et encore mieux de 0,1 à 30%.

Le milieu peut comporter une phase organique liquide dans laquelle de l'eau est dispersée ou émulsionnée.

La composition peut encore présenter une phase grasse continue, pouvant contenir moins de 5% d'eau, notamment moins de 1% d'eau par rapport à son poids total et en particulier être sous forme anhydre.

Agent filmogène

Le milieu peut comporter un agent filmogène, notamment un polymère filmogène.

On entend par « agent filmogène » un agent apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

La composition peut comporter une phase aqueuse et le polymère filmogène peut être présent dans cette phase aqueuse. Celui-ci pourra être un polymère en dispersion ou en solution.

La composition peut comporter une phase huileuse et le polymère filmogène peut être présent dans cette phase huileuse. Le polymère pourra alors être en dispersion ou en solution.

Parmi les polymères filmogènes utilisables, on peut citer les polymères synthétiques, de type radicalaire ou de type polycondensat, les polymères d'origine naturelle, et leurs mélanges.

Les polymères filmogènes de type radicalaire peuvent être notamment des polymères, ou des copolymères, vinyliques, notamment des polymères acryliques.

Parmi les polycondensats filmogènes, on peut citer les polyuréthanes, les polyesters, les polyesters amides, les polyamides, et les résines époxyesters, les polyurées.

Les polyesters peuvent être obtenus, de façon connue, par polycondensation d'acides dicarboxyliques avec des polyols, notamment des diols.

Les polyesters amides peuvent être obtenus de manière analogue aux polyesters, par polycondensation de diacides avec des diamines ou des amino alcools.

A titre d'exemple de polymères filmogènes liposolubles, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1

réticulé avec 0,2 % de divinyl benzène et propionate d'allyle/stéarate d'allyle réticulé avec 0,2 % de divinyl benzène.

Le polymère filmogène peut encore être choisi parmi les résines de silicone, généralement solubles ou gonflables dans les huiles de silicone, qui sont des polymères de polyorganosiloxanes réticulés.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Bien entendu, cette liste de polymères n'est pas exhaustive.

<u>Autres agents de coloration</u>

La composition peut comporter un ou plusieurs pigments diffusants, générant un couleur par un phénomène d'absorption, dans une proportion permettant de conserver le phénomène d'interférences responsable de la couleur de la composition une fois appliquée sur les matières kératiniques.

La composition peut ne pas comporter d'autres agents de coloration que le ou les pigment(s) interférentiel(s) multicouche, notamment être dépourvue de pigments à base d'oxydes de fer ou autres pigments générant une couleur par un phénomène d'absorption.

La composition peut contenir moins de 0,5% en masse de pigments générant une couleur par absorption, notamment moins de 0,5% de pigments à base d'oxydes de fer, mieux moins de 0,2%.

Quand un ou plusieurs pigments diffusants sont présents, divers pigments diffusants peuvent être envisagés, étant par exemple choisi parmi les laques ou pigments organiques sélectionnés notamment parmi les matériaux cidessous et leurs mélanges :

- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La laque peut être supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment

sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

Le pigment diffusant peut être un pigment composite, comportant un noyau enrobé au moins partiellement par une écorce. Un tel pigment composite peut être composé notamment de particules comportant un noyau inorganique et au moins un enrobage au moins partiel d'au moins une matière colorante organique. Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5. Un pigment composite peut présenter par exemple une surface spécifique comprise entre 1 et 1000 $m^2/g$, notamment entre 10 et 600 $m^2/g$ environ, et en particulier entre 20 et 400 $m^2/g$ environ. La surface spécifique est la valeur mesurée par la méthode BET. La proportion massique du noyau peut excéder 50 % par rapport au poids total du pigment composite, par exemple aller de 50 % à 70 %, par exemple de 60 % à 70 %.

L'agent de coloration peut encore être un colorant.

Il peut s'agir d'un colorant d'origine végétale, animale ou minérale, en particulier d'origine végétale ou minérale, notamment d'origine végétale. Ce colorant peut être de nature non synthétique.

Le colorant peut être un colorant naturel hydrosoluble ou liposoluble.

A titre illustratif des agents de coloration naturels hydrosolubles susceptibles d'être mis en oeuvre selon l'invention, on peut particulièrement citer le caramel, le jus de betterave et le carmin, la bétanine (betterave), la chlorophylline cuivrée, le bleu de méthylène, les anthocyanines (enocianine, carotte noire, hibiscus, sureau) et la riboflavine.

A titre illustratif des agents de coloration naturels liposolubles susceptibles d'être mis en oeuvre selon l'invention, on peut particulièrement citer le rouge Soudan, le β-carotène, les caroténoïdes, le lycopène, l'huile de palme, le brun Soudan, le jaune quinoléique les xanthophylles (capsanthine, capsorubine, lutéine), et le curcumin.

Comme autres colorants naturels, on peut plus particulièrement citer les anthocyanes de fleurs ou de fruits ou leurs dérivés, les flavonoïdes et tanins extraits de végétaux natifs ou fermentés, la juglone, la lawsone, les extraits de soja fermenté, d'algues, de champignons, de micro-organismes, les sels de Flavylium non substitués en position 3 tels que décrit dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

Comme exemple de colorants synthétiques, on peut citer les colorants liposolubles synthétiques tels que, par exemple, le DC Red 17, le DC Red 21, le DC Red 27, le DC Green 6, le DC Yellow 11, le DC Violet 2 et le DC Orange 5.

Comme exemple de colorants hydrosolubles synthétiques, on peut citer le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5 et le FDC Blue 1.

Charges

La composition cosmétique peut comprendre des charges.

Par « charges », on désigne des particules de toute forme insolubles dans le milieu de la composition, quelle que soit la température à laquelle la composition est fabriquée. Ces charges peuvent servir notamment à modifier la rhéologie ou la texture de la composition.

A titre d'exemple de charges, on peut citer, entre autres, le talc, le mica, la silice, le kaolin, et les poudres de polyamide (Nylon® ou Orgasol® de chez Atochem).

La teneur en charges sera choisie de façon à ne pas entraver outre mesure le résultat recherché.

Actifs et autres composés

La composition cosmétique peut également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, antirides...). Ces actifs peuvent être utilisés par exemple à des concentrations de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

La composition cosmétique peut, de plus, comprendre, selon le type d'application envisagée, les constituants classiquement utilisés dans les domaines considérés, qui sont présents en une quantité appropriée à la forme galénique souhaitée.

**Formes galéniques**

La composition cosmétique peut se présenter sous toute forme galénique normalement utilisée pour une application topique et notamment sous forme anhydre ou non anhydre, sous forme solide, pulvérulente libre ou compactée, coulée, par exemple en stick, liquide, d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules situés à l'interface huile/eau ou d'un spray.
Par « composition anhydre », on désigne une composition ayant moins de 5 % en masse d'eau, mieux moins de 3 %, voire moins de 1 %. Une composition anhydre peut ne pas comporter d'eau ajoutée intentionnellement au cours de la préparation de la composition.
La composition peut notamment se présenter sous forme de stick.
La composition cosmétique peut constituer, entre autres produits de maquillage, un rouge à lèvres en stick ou liquide, un brillant à lèvres liquide, une pâte de rouge à lèvres, un fard à joues, un fond de teint, un produit de contour des yeux, un eye-liner, un mascara, un vernis à ongles, une ombre à paupières, une base de maquillage, et plus généralement tout produit de maquillage du corps ou des cheveux.
La composition de l'invention peut être obtenue selon les procédés de préparation classiquement utilisés en cosmétique.

**Conditionnement et modes d'application**

La composition peut être conditionnée dans tout réceptacle ou sur tout support prévu à cet effet.
La composition peut être appliquée en utilisant un applicateur, floqué ou non, par exemple une mousse, un embout, un pinceau, un feutre, une spatule, un fritté, une brosse, un peigne, un tissé ou non tissé.
L'application peut encore s'effectuer avec le doigt ou en déposant directement la composition sur le support à maquiller, par exemple par friction dans le cas d'un stick ou par pulvérisation ou projection à l'aide d'un dispositif piézoélectrique ou par transfert d'une couche de composition préalablement déposée sur un support intermédiaire.
La composition peut, le cas échéant, être appliquée comme couche de base (base coat) recouverte d'une couche d'une autre composition (top coat), destinée par exemple à conférer de la brillance, ou comme couche de revêtement sur une couche d'une autre composition, voire entre une couche de base et une couche de revêtement.
La composition peut être conditionnée dans un dispositif de conditionnement qui laisse apparaître la couleur de la composition dans la masse.
Il peut s'agir d'un récipient ayant un corps au moins partiellement transparent et/ou d'un dispositif de conditionnement comportant un organe de bouchage au moins partiellement transparent.
La composition peut être conditionnée dans un dispositif qui permet de voir à la fois la couleur de la composition dans la masse et la couleur de la composition après application sur des matières kératiniques.
La composition peut encore être conditionnée dans un dispositif qui comporte des moyens représentatifs de la couleur révélée à l'application, par exemple un dépôt d'une couche de la composition ou d'une encre ou vernis comportant les mêmes agents de coloration que la composition.
Le conditionnement de la composition peut être agencé de manière à permettre d'exposer le ou les agents de coloration sensibles à un stimulus extérieur à ce stimulus, afin de pouvoir observer par exemple un changement de couleur de la composition dans la masse ou après application, lors de l'exposition au stimulus.
Par exemple, dans le cas d'agents de coloration photochromes, le conditionnement présente avantageusement une zone qui peut être couverte ou non, selon le choix du consommateur.
L'invention a encore pour objet une composition ainsi conditionnée.
La composition cosmétique, dans son conditionnement, peut présenter une zone qui peut être exposée ou non à la lumière.

**Procédé de maquillage**

L'invention a encore pour objet un procédé de maquillage des matières kératiniques au moyen d'une composition selon l'invention.
Il peut s'agir du maquillage de la peau, des lèvres, des ongles, des cils, sourcils ou cheveux.

**Procédé de présentation et présentoir**

L'invention a encore pour objet, selon un autre de ses aspects, un procédé de présentation d'une composition cosmétique réalisée conformément à l'invention, comportant les étapes consistant à :

- éclairer au moins une première région d'un échantillon de composition avec un éclairage diffus de façon à faire apparaître la couleur dans la masse,
- éclairer au moins une deuxième région d'un échantillon de composition avec un éclairage directionnel de façon à faire apparaître la couleur interférentielle.

Les première et deuxième régions peuvent être des régions différentes d'un même échantillon de composition.
En variante, les première et deuxième régions appartiennent à des échantillons différents.
En variante encore, les première et deuxième régions correspondent à une même région et les éclairages diffus et directionnel sont appliqués alternativement.
L'invention a encore pour objet, selon un autre de ses aspects, un présentoir comportant :

- une source d'éclairage directionnel,
- une source d'éclairage diffus,
- un support pour permettre d'exposer au moins un échantillon de composition à la source d'éclairage diffus et au moins un échantillon de composition à la source d'éclairage directionnel.

Le présentoir peut être agencé pour permettre d'exposer le ou les agents de coloration sensibles à un stimulus extérieur à ce stimulus, afin de mettre en évidence la variation de couleur correspondante.
Dans l'exemple d'un agent de coloration thermochrome, le présentoir est par exemple agencé pour exposer un échantillon de composition à une source de chaleur afin de provoquer un changement d'état.
Le présentoir comporte par exemple un support chauffé sur lequel la composition est étalée afin d'une part de révéler la couleur du ou des pigments interférentiels multicouche à l'application et d'autre part d'induire un changement d'état de l'agent de coloration thermochrome.
Dans le cas d'un agent de coloration photochrome, l'éclairage utilisé pour éclairer la composition afin de révéler la couleur du pigment interférentiel multicouche peut comporter des UV afin d'induire un changement d'état de l'agent de coloration photochrome.

**Kit**

La présente invention a également pour objet un kit de maquillage comportant :

- une première composition selon l'invention,
- une deuxième composition comportant un milieu cosmétiquement acceptable et à appliquer sous ou sur la première composition.

Cette deuxième composition est par exemple destinée à améliorer la tenue de la première composition et/ou à en modifier l'aspect.

**Exemples proposés**

Les exemples 1 à 7 concernent des compositions comportant au moins un pigment interférentiel multicouche et au moins un agent de coloration Xchrome.
Les teneurs indiquées sont massiques.

Exemple 1 : Brillant à lèvres

| | |
|---|---|
| Octyl-2 dodécanol | 0 |
| Ditertiobutyl 4-hydroxytoluène | ,07 |
| Polybutène (monooléfines / isoparaffines 95/5) (PM : 2060) | 7 |

(suite)

| | |
|---|---|
| Mélange de p-hydroxybenzoates d'isopropyle, iso-butyle, n-butyle (40/30/30) | ,6 |
| Tetra-iso-stéarate de pentaérythrityle | 1,33 |
| Tri-mellitate de tridécyle | 1 |
| Triglycéride d'acide 2-décyl tétradécanoïque (GUERBET C24) | 5 |
| Pigment interférentiel multicouche* | 2 |
| Agent de coloration Xchrome** | |
| * TIMIRON SILK RED de la société MERCK<br>** REVERSACOL de la société JAMES ROBINSON | |

L'effet produit par ce brillant à lèvres résulte de l'association de plusieurs phénomènes. Le premier concerne le rendu coloriel à l'application qui est intense et couvrant. A cela s'ajoute l'effet Xchrome qui induira un changement de couleur selon le type de lumière et l'exposition suivie. Ce brillant à lèvres présente une couleur de masse qui est blanche.

Exemple 2 : Rouge à lèvres

| | |
|---|---|
| Tri-mellitate de tri-décyle | 1 |
| Lanoline liquide | 0 |
| Malate d'iso-stéaryle | 2 |
| Lanoline acétylée | 0 |
| Triglycérides d'acides laurique/palmitique/cétylique/stéarique (50/20/10/10) | |
| Cire microcrystalline (C20-C60) | |
| Lanolate d'isopropyle protégé | |
| Octyl-2-décanol | 5 |
| Phényl triméthylsiloxy trisiloxane (VISCOSITE: 20 CST - PM: 372) | |
| Cire de polyéthylène (PM :500) | |
| Pigment interférentiel multicouche* | 0 |
| Agent de coloration Xchrome** | |
| * TIMIRON SILK RED de la société MERCK<br>** REVERSACOL de la société JAMES ROBINSON | |

L'effet produit par ce rouge à lèvres résulte de l'association de plusieurs phénomènes. Le premier concerne le rendu coloriel à l'application qui est intense et couvrant. A cela s'ajoute l'effet Xchrome qui induira un changement de couleur selon le type de lumière et l'exposition suivie. Ce rouge à lèvres présente une couleur de masse qui est blanche.

Exemple 3 : Blush

| | |
|---|---|
| Triéthano lamine | |
| Acide éthylène diamine tetracétique, sel disodique, 2H2O | ,2 |
| Homopolymère carboxyvinylique réticulée | ,5 |
| Polyvinylpyrro lidone | ,6 |

(suite)

| | |
|---|---|
| Triéthano lamine | |
| Glycérol | ,75 |
| Eau désionisée | 7,45 |
| 1,3 butylène glycol | |
| Microsphère de silice (3µm) | ,5 |
| Pigment interférentiel multicouche* | |
| Agent de coloration Xchrome* * | |
| * TIMIRON SILK RED de la société MERCK<br>** Photogenica® de la société CATALYST & CHEMICALS | |

L'effet produit par ce blush résulte de l'association de plusieurs phénomènes. Le premier concerne le rendu coloriel à l'application qui est intense et couvrant. A cela s'ajoute l'effet Xchrome qui induira un changement de couleur selon le type de lumière et l'exposition suivie. Ce blush présente une couleur de masse qui est blanche.

Exemple 4 : Vernis à ongles à base aqueuse

| | |
|---|---|
| Pyophosphate tétrasodique | ,2 |
| Polydiméthylsiloxane oxyéthylène à terminaisons méthoxy | ,5 |
| Mélange de polyuréthane aliphatique, N-méthyl pyrrolidone, triéthylamine,eau (35/8,5/2/54,5) | 5 |
| Glycérol | |
| Eau désionisée | 5 |
| Alcool éthylique (96°) | ,8 |
| Laponite synthétique (silicate mixte magnésium/lithium/sodium) | ,3 |
| Pigment interférentiel multicouche** | 1,2 |
| Agent de coloration Xchrome** | |
| * TIMIRON SILK RED de la société MERCK<br>** Kromafast® Yellow5GX 02 de la société KROMACHEM LTD | |

L'effet produit par ce vernis à ongles résulte de l'association de plusieurs phénomènes. Le premier concerne le rendu coloriel à l'application qui est intense et couvrant. A cela s'ajoute l'effet Xchrome qui induira un changement de couleur selon le type de lumière et l'exposition suivie. Ce vernis à ongles présente une couleur de masse qui est blanche.

Exemple 5 : Vernis à ongles à base solvant organique

| | |
|---|---|
| Nitrocellulose | 1 |
| N-éthyl o,p-toluènesulfonamide | |
| Résine alkyde | 0 |
| Isopropanol | |
| Pigment interférentiel multicouche* | 0 |
| Agent de coloration Xchrome** | |

(suite)

| | |
|---|---|
| Acétate de butyle/acétate d'éthyle 50/50 | sp 100 |
| * TIMIRON SILK RED de la société MERCK<br>** Kromafast® Yellow5GX 02 de la société KROMACHEM LTD | |

L'effet produit par ce vernis à ongles résulte de l'association de plusieurs phénomènes. Le premier concerne le rendu coloriel à l'application qui est intense et couvrant. A cela s'ajoute l'effet Xchrome qui induira un changement de couleur selon le type de lumière et l'exposition suivie. Ce vernis à ongles présente une couleur de masse qui est blanche.

<u>Exemple 6</u> : <u>Fard à paupière en poudre</u>

| | |
|---|---|
| TERTIOBUTYL 4-HYDROXYANISOLE | ,012 |
| DITERTIOBUTYL 4-HYDROXYTOLUENE | ,012 |
| VASELINE BLANCHE | ,2 |
| ALCOOL OLEIQUE | ,2 |
| LANOLINE LIQUIDE PROTEGEE | ,66 |
| HUILE DE VASELINE | ,516 |
| HUILE DE RICIN | ,296 |
| MYRISTATE D'ISOPROPYLE | ,864 |
| P-HYDROXYBENZOATE DE PROPYLE | ,24 |
| MELANGE P-HYDROXYBENZOATES DE METHYLE, ETHYLE, PROPYLE, BUTYLE ISOBUTYLE / PHENOXY-2 ETHANOL | ,6 |
| Pigment interférentiel multicouche* | 0,3 |
| STEARATE DE MAGNESIUM | |
| TALC | 0,1 |
| Matériau à propriétés Xchromiques** | |
| * TIMIRON SILK RED de la société MERCK<br>** Photogenica® de la société CATALYST & CHEMICALS | |

L'effet produit par fard à paupières résulte de l'association de plusieurs phénomènes. Le premier concerne le rendu coloriel à l'application qui est intense et couvrant. A cela s'ajoute l'effet Xchrome qui induira un changement de couleur selon le type de lumière et l'exposition suivie. Ce fard à paupières présente une couleur de masse qui est blanche.

<u>Exemple 7</u> : <u>Fard à paupière liquide</u>

| | |
|---|---|
| DITERTIOBUTYL 4-HYDROXYTOLUENE | 0,09 |
| HECTORITE MODIFIEE DISTEARYL DIMETHYL AMMONIUM | 3,74 |
| TRIGLYCERIDES D'ACIDES LAURIQUE/PALMITIQUE/CETYLIQUE/STEARIQUE (50/20/10/10) | 6,46 |
| CARBONATE DE PROPYLENE | 1,22 |
| CIRE D'ABEILLE BLANCHIE | 7,77 |

(suite)

| | |
|---|---|
| BEURRE DE SHOREA PROTEGE | 1,7 |
| FRACTION LIQUIDE DE BEURRE DE KARITE PROTEGEE | 0,85 |
| POUDRE DE NYLON 12 | 10,4 |
| ISO-DODECANE | 35,32 |
| P-HYDROXYBENZOATE DE PROPYLE | 0,17 |
| PARAFFINE RAFFINEE PROTEGEE | 3,88 |
| TALC | 10,4 |
| Pigment interférentiel multicouche* | 15 |
| Matériaux à propriétés Xchromiques** | 3 |
| * TIMIRON SILK RED de la société MERCK<br>** Photogenica® de la société CATALYST & CHEMICALS | |

L'effet produit par fard à paupières résulte de l'association de plusieurs phénomènes. Le premier concerne le rendu coloriel à l'application qui est intense et couvrant. A cela s'ajoute l'effet Xhcrome qui induira un changement de couleur selon le type de lumière et l'exposition suivie. Ce fard à paupières présente une couleur de masse qui est blanche.

Les exemples 8 à 14 concernent des compositions comportant au moins un pigment interférentiel multicouche et au moins un pigment réfléchissant à reflet métallique.

Exemple 8 : Brillant à lèvres

| | |
|---|---|
| Octyl-2 dodécanol | 0 |
| Ditertiobutyl 4-hydroxytoluene | ,07 |
| Polybutène (monooléfines / isoparaffines 95/5) (PM : 2060) | 7 |
| Mélange de p-hydroxybenzoates d'isopropyle, iso-butyle, n-butyle (40/30/30) | ,6 |
| Tetra-iso-stéarate de pentaérythrityle | 1,33 |
| Tri-mellitate de tridécyle | 1 |
| Triglycéride d'acide 2-décyl tétradécanoïque (GUERBET C24) | 5 |
| Pigment interferentiel multicouche * | 2 |
| Pigment à réflexion métallique ** | |
| * TIMIRON SILK BLUE de la société MERCK<br>** *METASHINE ME 2040 PS* de la société NIPPON SHEET GLASS | |

L'effet produit par ce brillant à lèvres résulte de l'association de plusieurs phénomènes. Le premier concerne le rendu coloriel à l'application qui est intense et couvrant. A cela s'ajoutent des points de brillance apportés par les particules à reflet métallique. Ce brillant à lèvres présente une couleur de masse qui est blanche.

Exemple 9 : Rouge à lèvres

| | |
|---|---|
| Tri-mellitate de tri-decyle | 1 |
| Lanoline liquide | 0 |
| Malate de d'iso-stearyle | 2 |

(suite)

| | |
|---|---|
| Lanoline acetylée | 0 |
| Triglycerides d'acides laurique/palmitique/cetylique/stearique (50/20/10/10) | |
| Cire microcrystalline (C20-C60) | |
| Lanolate d'isopropyle protégé | |
| Octyl-2-decanol | 5 |
| Phényl trimethylsiloxy trisiloxane (VISCOSITE: 20 CST - PM: 372) | |
| Cire de polyéthylène (PM :500) | |
| Pigment interférentiel multicouche * | 0 |
| Pigment à réflexion métallique** | |
| * TIMIRON SILK RED de la société MERCK<br>** *METASHINE ME 2040 PS* de la société NIPPON SHEET GLASS | |

L'effet produit par ce rouge à lèvres résulte de l'association de plusieurs phénomènes. Le premier concerne le rendu coloriel à l'application qui est intense et couvrant. A cela s'ajoutent des points de brillance apportés par les particules à reflet métallique. Ce rouge à lèvres présente une couleur de masse qui est blanche.

Exemple 10 : Blush

| | |
|---|---|
| Triéthano lamine | |
| Acide éthylène diamine tetracétique, sel disodique, $2H_2O$ | ,2 |
| Homopolymère carboxyvinylique réticulée | ,5 |
| Polyvinylpyrro lidone | ,6 |
| Glycérol | ,75 |
| Eau désionisée | 7,45 |
| 1,3 butylène glycol | |
| Microsphère de silice (3µm) | ,5 |
| Pigment interférentiel multicouche * | |
| Pigment à réflexion métallique ** | |
| * TIMIRON SILK RED de la société MERCK<br>** *METASHINE ME 2040 PS* de la société NIPPON SHEET GLASS | |

L'effet produit par ce blush résulte de l'association de plusieurs phénomènes. Le premier concerne le rendu coloriel à l'application qui est intense et couvrant. A cela s'ajoutent des points de brillance apportés par les particules à reflet métallique. Ce blush présente une couleur de masse qui est blanche.

Exemple 11 : Vernis à ongles à base aqueuse

| | |
|---|---|
| Pyophosphate tétrasodique | 0,2 |
| Polydimethylsiloxane oxyethylène à terminaisons méthoxy | 0,5 |
| Mélange de polyuréthane aliphatique, N-méthyl pyrrolidone, triéthylamine,eau (35/8,5/2/54,5) | 65 |

(suite)

| | |
|---|---|
| Glycérol | 1 |
| Eau désionisée | 15 |
| Alcool éthylique (96°) | 2,8 |
| Laponite synthétique (silicate mixte magnésium/lithium/sodium) | 1,3 |
| Pigment interférentiel multicouche * | 11,2 |
| Pigment à réflexion métallique** | 3 |
| * TIMIRON SILK RED de la société MERCK<br>** *METASHINE ME 2040 PS* de la société NIPPON SHEET GLASS | |

Exemple 12 : Vernis à ongles à base solvant organique

| | |
|---|---|
| Nitrocellulose | 1 |
| N-éthyl o,p-toluènesulfonamide | |
| Résine alkyde | 0 |
| Isopropanol | |
| Pigment interférentiel multicouche* | 0 |
| Pigment à réflexion métallique ** | |
| Acétate de butyle/acétate d'éthyle 50/50 | Qsp 100 |
| * XIRONA SILVER de la société MERCK<br>** *METASHINE ME 2040 PS* de la société NIPPON SHEET GLASS | |

Les particules à reflet métallique apportent des points de brillance. Ce vernis à ongles présente une couleur de masse qui est blanche.

Exemple 13 : Fards à paupière en poudre

| | |
|---|---|
| TERTIOBUTYL 4-HYDROXYANISOLE | 0,012 |
| DITERTIOBUTYL 4-HYDROXYTOLUENE | 0,012 |
| VASELINE BLANCHE | 1,2 |
| ALCOOL OLEIQUE | 1,2 |
| LANOLINE LIQUIDE PROTEGEE | 0,66 |
| HUILE DE VASELINE | 6,516 |
| HUILE DE RICIN | 1,296 |
| MYRISTATE D'ISOPROPYLE | 0,864 |
| P-HYDROXYBENZOATE DE PROPYLE | 0,24 |
| MELANGE P-HYDROXYBENZOATES DE METHYLE, ETHYLE, PROPYLE, BUTYLE ISOBUTYLE / PHENOXY-2 ETHANOL | 0,6 |
| pigment interférentiel multicouche* | 50,3 |

(suite)

| | |
|---|---|
| STEARATE DE MAGNESIUM | 4 |
| TALC | 30,1 |
| Pigment à réflexion métallique** | 3 |
| * TIMIRON SILK RED de la société MERCK<br>** *METASHINE ME 2040 PS* de la société NIPPON SHEET GLASS | |

Exemple 14 : Fards à paupière liquide

| | |
|---|---|
| DITERTIOBUTYL 4-HYDROXYTOLUENE | ,09 |
| HECTORITE MODIFIEE DISTEARYL DIMETHYL AMMONIUM | ,74 |
| TRIGLYCERIDES D'ACIDES LAURIQUE/PALMITIQUE/CETYLIQUE/STEARIQUE (50/20/10/10) | ,46 |
| CARBONATE DE PROPYLENE | ,22 |
| CIRE D'ABEILLE BLANCHIE | ,77 |
| BEURRE DE SHOREA PROTEGE | ,7 |
| FRACTION LIQUIDE DE BEURRE DE KARITE PROTEGEE | ,85 |
| POUDRE DE NYLON 12 | 0,4 |
| ISO-DODECANE | 5,32 |
| P-HYDROXYBENZOATE DE PROPYLE | ,17 |
| PARAFFINE RAFFINEE PROTEGEE | ,88 |
| TALC | 0,4 |
| pigment interférentiel multicouche* | 5 |
| Pigment à réflexion métallique ** | |
| ** TIMIRON SILK RED de la société MERCK*<br>*** METASHINE ME 2040 PS de la société NIPPON SHEET GLASS* | |

Le fond est de préférence bien étalé afin de révéler l'effet coloriel. Un embout floqué peut être utilisé à cet effet.

Les exemples 15 à 21 concernent des compositions comportant au moins un pigment interférentiel multicouche et au moins un pigment diffractant.

Exemple 15 : Brillant à lèvres

| | |
|---|---|
| Octyl-2 dodécanol | 0 |
| Ditertiobutyl 4-hydroxytoluène | ,07 |
| Polybutène (monooléfines / isoparaffines 95/5) (PM : 2060) | 7 |
| Mélange de p-hydroxybenzoates d'isopropyle, iso-butyle, n-butyle (40/30/30) | ,6 |
| Tétra-iso-stéarate de pentaérythrityle | 1,33 |
| Tri-mellitate de tridécyle | 1 |
| Triglycéride d'acide 2-décyl tétradécanoïque (GUERBET C24) | 5 |
| Pigment interférentiel multicouche* | 2 |

(suite)

| | |
|---|---|
| Pigment diffractant** | |
| ** TIMIRON SILK RED de la société MERCK<br>** Pigment diffractant SPECTRAFLAIR de la société FLEXPRODUCTS* | |

L'effet produit par le brillant à lèvres résulte de l'association de plusieurs phénomènes. Le premier concerne le rendu coloriel à l'application qui est intense et couvrant. A cela s'ajoute l'effet « arc-en-ciel » apporté par le pigment diffractant. Ce fard à paupières présente une couleur de masse qui est blanche.

Exemple 16 : Rouge à lèvres

| | |
|---|---|
| Tri-mellitate de tri-décyle | 1 |
| Lanoline liquide | 0 |
| Malate d'iso-stéaryle | 2 |
| Lanoline acétylée | 0 |
| Triglycérides d'acides laurique/palmitique/cétylique/stéarique (50/20/10/10) | |
| Cire microcristalline (C20-C60) | |
| Lanolate d'isopropyle protégé | |
| Octyl-2-decanol | 5 |
| Phényl triméthylsiloxy trisiloxane (VISCOSITE: 20 CST - PM: 372) | |
| Cire de polyéthylène (PM :500) | |
| Pigment interférentiel multicouche* | 0 |
| Pigment diffractant** | |
| ** TIMIRON SILK RED de la société MERCK<br>** Pigment diffractant SPECTRAFLAIR de la société FLEXPRODUCTS* | |

L'effet produit par ce rouge à lèvres résulte de l'association de plusieurs phénomènes. Le premier concerne le rendu coloriel à l'application qui est intense et couvrant. A cela s'ajoute l'effet « arc-en-ciel » apporté par le pigment diffractant. Ce rouge à lèvres présente une couleur de masse qui est blanche.

Exemple 17 : Blush

| | |
|---|---|
| Triéthano lamine | |
| Acide éthylène diamine tétracétique, sel disodique, 2H2O | ,2 |
| Homopolymère carboxyvinylique réticulée | ,5 |
| Polyvinylpyrro lidone | ,6 |
| Glycérol | ,75 |
| Eau désionisée | 7,45 |
| 1,3 butylène glycol | |
| Microsphère de silice (3μm) | ,5 |
| Pigment interférentiel multicouche* | |

(suite)

| | |
|---|---|
| Pigment diffractant** | |
| * TIMIRON SILK RED de la société MERCK<br>** Pigment diffractant SPECTRAFLAIR de la société FLEX PRODUCTS | |

L'effet produit par ce blush résulte de l'association de plusieurs phénomènes. Le premier concerne le rendu coloriel à l'application qui est intense et couvrant. A cela s'ajoute l'effet « arc-en-ciel » apporté par le pigment diffractant. Ce blush présente une couleur de masse qui est blanche.

Exemple 18 : Vernis à ongles à base aqueuse

| | |
|---|---|
| Pyophosphate tétrasodique | ,2 |
| Polydiméthylsiloxane oxyéthylène à terminaisons méthoxy | ,5 |
| Mélange de polyuréthane aliphatique, N-méthyl pyrrolidone, triéthylamine, eau (35/8,5/2/54,5) | 5 |
| Glycérol | |
| Eau désionisée | 5 |
| Alcool éthylique (96°) | ,8 |
| Laponite synthétique (silicate mixte magnésium/lithium/sodium) | ,3 |
| Pigment interférentiel multicouche* | 1,2 |
| Pigment diffractant** | |
| * TIMIRON SILK BLUE de la société MERCK<br>** Pigment diffractant SPECTRAFLAIR de la société FLEX PRODUCTS | |

L'effet produit par ce vernis à ongles résulte de l'association de plusieurs phénomènes. Le premier concerne le rendu coloriel à l'application qui est intense et couvrant. A cela s'ajoute l'effet « arc-en-ciel » apporté par le pigment diffractant. Ce vernis à ongles présente une couleur de masse qui est blanche.

Exemple 19 : Vernis à ongles à base solvant organique

| | |
|---|---|
| Nitrocellulose | 1 |
| N-éthyl o,p-toluènesulfonamide | |
| Résine alkyde | 0 |
| Isopropanol | |
| Pigment interférentiel multicouche* | 0 |
| Pigment diffractant** | |
| Acétate de butyle/acétate d'éthyle 50/50 | Qsp 100 |
| * XIRONA SILVER de la société MERCK<br>** Pigment diffractant SPECTRAFLAIR de la société FLEX PRODUCTS | |

L'effet produit par ce vernis à ongles résulte de l'association de plusieurs phénomènes. Le premier concerne le rendu coloriel à l'application qui est intense et couvrant. A cela s'ajoute l'effet « arc-en-ciel » apporté par le pigment

diffractant. Ce vernis à ongles présente une couleur de masse qui est blanche.

Exemple 20 : Fard à paupière en poudre

| | |
|---|---|
| TERTIOBUTYL 4-HYDROXYANISOLE | ,012 |
| DITERTIOBUTYL 4-HYDROXYTOLUENE | ,012 |
| VASELINE BLANCHE | ,2 |
| ALCOOL OLEIQUE | ,2 |
| LANOLINE LIQUIDE PROTEGEE | ,66 |
| HUILE DE VASELINE | ,516 |
| HUILE DE RICIN | ,296 |
| MYRISTATE D'ISOPROPYLE | ,864 |
| P-HYDROXYBENZOATE DE PROPYLE | ,24 |
| MELANGE P-HYDROXYBENZOATES DE METHYLE, ETHYLE, PROPYLE, BUTYLE ISOBUTYLE / PHENOXY-2 ETHANOL | ,6 |
| Pigment interférentiel multicouche* | 0,3 |
| STEARATE DE MAGNESIUM | |
| TALC | 0,1 |
| Pigment difïractant** | |
| * TIMIRON SILK RED de la société MERCK<br>** Pigment diffractant SPECTRAFLAIR de la société FLEX PRODUCTS | |

L'effet produit par ce fard à paupière résulte de l'association de plusieurs phénomènes. Le premier concerne le rendu coloriel à l'application qui est intense et couvrant. A cela s'ajoute l'effet « arc-en-ciel » apporté par le pigment diffractant. Ce vernis à ongles présente une couleur de masse qui est blanche.

Exemple 21 : Fard à paupière liquide

| | |
|---|---|
| DITERTIOBUTYL 4-HYDROXYTOLUENE | ,09 |
| HECTORITE MODIFIEE DISTEARYL DIMETHYL AMMONIUM | ,74 |
| TRIGLYCERIDES D'ACIDES LAURIQUE/PALMITIQUE/CETYLIQUE/STEARIQUE (50/20/10/10) | ,46 |
| CARBONATE DE PROPYLENE | ,22 |
| CIRE D'ABEILLE BLANCHIE | ,77 |
| BEURRE DE SHOREA PROTEGE | ,7 |
| FRACTION LIQUIDE DE BEURRE DE KARITE PROTEGEE | ,85 |
| POUDRE DE NYLON 12 | 0,4 |
| ISO-DODECANE | 2,32 |
| P-HYDROXYBENZOATE DE PROPYLE | ,17 |
| PARAFFINE RAFFINEE PROTEGEE | ,88 |
| TALC | 0,4 |
| Pigment interférentiel multicouche* | 5 |

(suite)

| | |
|---|---|
| Pigment diffractant** | |
| * TIMIRON SILK RED de la société MERCK<br>** Pigment diffractant SPECTRAFLAIR de la société FLEX PRODUCTS | |

L'effet produit par ce fard à paupière résulte de l'association de plusieurs phénomènes. Le premier concerne le rendu coloriel à l'application qui est intense et couvrant. A cela s'ajoute l'effet « arc-en-ciel » apporté par le pigment diffractant. Ce vernis à ongles présente une couleur de masse qui est blanche.

Ensemble pour application bi-couche

On peut associer la composition selon l'invention à une composition de recouvrement ou de base. On décrit ci-dessous des exemples 22 et 23 d'associations. Dans les exemples 22 et 23, l'une des première et deuxième composition peut encore être remplacée par l'une des compositions des exemples 1 à 21.

Exemple 22 : Rouge à lèvres

Première composition

| | |
|---|---|
| Tri-mellitate de tri-décyle | 11 |
| Lanoline liquide | 10 |
| Malate d'iso-stéaryle | 13 |
| Lanoline acétylène | 10 |
| Triglycérides d'acides laurique/palmitique/cétylique/stéarique (50/20/10/10) | 5 |
| Cire microcristalline (C20-C60) | 3 |
| Lanolate d'isopropyle protégé | 10 |
| Octyl-2-decanol | 16 |
| Phényl triméthylsiloxy trisiloxane (VISCOSITE: 20 CST - PM: 372) | 4 |
| Cire de polyéthylène (PM :500) | 8 |
| Pigment interférentiel multicouche* | 10 |
| * TIMIRON SILK RED de la société MERCK | |

Deuxième composition (de recouvrement)

| | |
|---|---|
| Octyl-2 dodécanol | 0 |
| Ditertiobutyl 4-hydroxytoluène | ,07 |
| Polybutène (monooléfines / isoparaffines 95 / 5) (PM : 2060) | 0 |
| Mélange de p-hydroxybenzoates d'isopropyle, iso-butyle, n-butyle (40/30/30) | ,4 |
| Tétra-iso-stéarate de pentaérythrityle | 1,33 |
| Tri-mellitate de tridécyle | 2 |
| Triglycéride d'acide 2-décyl tétradécanoïque (GUERBET C24) | 1 |
| Pigment à effet** | |
| ** METASHINE ME 2040 PS de la société NIPPON SHEET GLASS | |

La première composition permet de générer une couche de maquillage homogène d'une couleur rouge très intense. La seconde composition permet de créer un effet loupe agrémenté de point de surbrillance argent qui donnent

du relief à l'ensemble du résultat maquillage.

Exemple 23 : Rouge à lèvres

Composition de base

| | |
|---|---|
| Tri-mellitate de tri-décyle | 12 |
| Lanoline liquide | 11 |
| Malate de d'iso-stéaryle | 14 |
| Lanoline acétylène | 11 |
| Triglycérides d'acides laurique/palmitique/cétylique/stéarique (50/20/10/10) | 6 |
| Cire microcristalline (C20-C60) | 3 |
| Lanolate d'isopropyle protégé | 10 |
| Octyl-2-decanol | 16 |
| Phényl triméthylsiloxy trisiloxane (VISCOSITE: 20 CST - PM: 372) | 4 |
| Cire de polyéthylène (PM :500) | 8 |
| Pigment noir d'oxyde de Fer $Fe_3O_4$ | 5 |

Composition contenant le pigment interférentiel multicouche

| | |
|---|---|
| Octyl-2 dodécanol | 10 |
| Ditertiobutyl 4-hydroxytoluène | 0,07 |
| Polybutène (monooléfines / isoparaffines 95 / 5) (PM : 2060) | 45,2 |
| Mélange de p-hydroxybenzoates d'isopropyle, iso-butyle, n-butyle (40/30/30) | 0,4 |
| Tétra-iso-stéarate de pentaérythrityle | 11,33 |
| Tri-mellitate de tridécyle | 12 |
| Triglycéride d'acide 2-décyl tétradécanoïque (GUERBET C24) | 11 |
| Pigment interférentiel multicouche ** | 10 |
| ** TIMIRON SILK RED de la société MERCK | |

La composition de base est appliquée en premier et permet de générer une couche de maquillage homogène d'une couleur noire très intense. La composition contenant le pigment interférentiel multicouche est appliquée ensuite et permet de créer un effet coloré donnant un résultat maquillage très intense.
La description suivant concerne un autre aspect de l'invention.

Stick multicolore

Mesure de la couvrance du produit

Les compositions du produit solide décrit plus haut sont fondues et mélangées, puis la couvrance est mesurée comme ci-dessus pour une composition unique.

Composé pâteux

Le milieu cosmétiquement acceptable peut comporter un composé pâteux choisi par exemple parmi :

- les composés siliconés polymères ou non,
- les composés fluorés polymères ou non,

- les polymères vinyliques, notamment:

  - les homopolymères d'oléfines
  - les copolymères d'oléfines
  - les homopolymères et copolymères de diènes hydrogénés
  - les oligomères linéaires ou ramifiés, homo ou copolymères de (méth)acrylates d'alkyles ayant de préférence un groupement alkyle en $C_8$-$C_{30}$
  - les oligomères homo et copolymères d'esters vinyliques ayant des groupements alkyles en $C_8$-$C_{30}$
  - les oligomères homo et copolymères de vinyléthers ayant des groupements alkyles en $C_8$-$C_{30}$,

- les polyéthers liposolubles résultant de la polyéthérification entre un ou plusieurs diols en $C_2$-$C_{100}$, de préférence en $C_2$-$C_{50}$,
- les esters,
- et leurs mélanges.

Le composé pâteux est de préférence un polymère, notamment hydrocarboné.

Composés pâteux siliconés et /ou fluorés

Un exemple de composé pâteux siliconé et fluoré est le polyméthyl trifluoropropryl méthylalkyl diméthylsiloxane, fabriqué sous la dénomination X22-1088 par la société SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Composés pâteux polyéthers

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéariques, à l'image notamment de ceux commercialisés sous la marque Softisan 649 par la société Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en $C_4$-$C_{50}$ et un diol ou un polyol en $C_2$-$C_{50}$,
- les esters aliphatiques d'ester résultant de l'estérification d'un ester d'acide hydroxycarboxylique aliphatique par un acide carboxylique aliphatique; et leurs mélanges.

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

L'acide carboxylique aliphatique est de préférence ramifié.

L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :

a) les esters partiels ou totaux de monoacides carboxyliques aliphatiques mono hydroxylés linéaires, saturés ;
b) les esters partiels ou totaux de monoacides carboxyliques aliphatiques mono hydroxylés insaturés ;

c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en $C_2$ à $C_{16}$ ayant réagi avec un mono ou un poly acide carboxylique aliphatique mono ou poly hydroxylé,

et leurs mélanges.
Les esters aliphatiques d'ester sont avantageusement choisis parmi :

- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.
Le composé pâteux représente de préférence 1 à 99%, mieux 1 à 60%, mieux 2 à 30% et mieux encore 5 à 15% en poids de chaque composition du produit solide.
En particulier, un composé pâteux et un ester tels que définis ci-dessus peuvent être associés dans la composition cosmétique dans un rapport pondéral composé pâteux/ester variant de 0,25 à 0,75 notamment de 0, 3 à 0,6.
La composition cosmétique selon l'invention peut présenter une phase grasse continue, pouvant contenir moins de 5 % d'eau, notamment moins de 1 % d'eau par rapport à son poids total.

Phase grasse

Chaque composition, notamment lorsque le produit est destiné à être appliqué sur les lèvres, peut comporter au moins une phase grasse et notamment au moins un corps gras liquide à température ambiante (25 °C) et à pression atmosphérique et/ou un corps gras solide à température ambiante et à pression atmosphérique tel que les cires, les gommes et leurs mélanges.
La phase grasse peut en outre contenir des agents gélifiants et structurants d'huiles de nature organique et/ou des solvants organiques lipophiles.
La phase grasse de la composition selon l'invention peut comprendre, à titre de corps gras liquide, au moins une huile volatile ou non volatile ou un de leurs mélanges.
Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).
Par "huile non volatile", on entend une huile restant sur la peau à température ambiante et pression atmosphérique au moins plusieurs heures et ayant notamment une pression de vapeur inférieure à 0,01 mm de Hg (1,33 Pa).
Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.
Les huiles hydrocarbonées volatiles peuvent être choisies parmi les huiles hydrocarbonées ayant de 8 à 16 atomes de carbone, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls®, les esters ramifiés en C8-C16 tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.
Comme huiles volatiles, on peut aussi utiliser les silicones volatiles, comme par exemple les huiles de silicones linéaires ou cycliques volatiles, notamment celles ayant une viscosité ≤ 8 centistokes (8 x 10-6 m2/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,1 % à 98 % en poids, notamment de 1 % à 65 % en poids, et en particulier de 2 % à 50 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 1810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans une composition d'un produit selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates et leurs mélanges.

Les huiles non volatiles peuvent être présentes dans une composition d'un produit selon l'invention en une teneur allant de 0,01 à 90 % en poids, notamment de 0,1 % à 85% en poids, et en particulier de 1 % à 70 % en poids, par rapport au poids total de la composition.

Les huiles peuvent représenter de 0 à 99 % du poids total de chaque composition, de préférence de 0,05 à 60 % et mieux de 1 à 35 %.

Les huiles peuvent être de poids moléculaire compris entre 650 et 10.000 g/mol, de préférence entre 750 et 7.500 g/mol.

Selon un mode de mise en oeuvre, chaque composition comprend une phase huileuse comprenant au moins 70 % en poids d'une huile de masse molaire comprise entre 650 et 10.000 g/mol, de préférence entre 750 et 7.500 g/mol. La phase huileuse comprend avantageusement plus de 80 %, de préférence plus de 85 % en poids d'un huile de masse molaire comprise entre 650 et 10.000 g/mol, de préférence entre 750 et 7.500 g/mol.

L'huile de masse molaire élevée peut être choisie parmi les polymères lipophiles :

- les esters d'acides gras linéaires ayant un nombre total de carbones allant de 35 à 70,
- les esters hydroxylés,
- les esters aromatiques,
- les esters d'alcool gras ou d'acides gras ramifiés en C24-C28,

- les huiles siliconées,
- les huiles d'origine végétale,
- et leurs mélanges.

L'huile de masse molaire élevée peut être choisie parmi les polybutylènes, les polyisobutylènes hydrogénés, les polydécènes, les polydécènes hydrogénés, les copolymères de la vinylpyrrolidone tel que le PVP / héxadécène copolymère, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de glycéryle, le tétraisostéarate de pentaérythrityle, le tri décyl-2 tétradécanoate de glycéryle, le tétra décyl-2 tétradécanoate de pentaérythrityle, les silicones phénylées, l'huile de sésame, et leurs mélanges.

Chaque composition peut également comporter un corps gras solide à température ambiante et à pression atmosphérique, choisi par exemple parmi les cires, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Cires et gommes

Chaque composition peut contenir au moins une cire.

Par "cire", on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40 °C et mieux supérieure à 45 °C.

Comme cire utilisable, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxydiméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

Les gommes utilisables dans l'invention se présentent généralement sous forme solubilisée dans une huile, les polymères sont solides à température ambiante et les résines peuvent être liquides ou solides à température ambiante.

Par « gomme » on entend un corps gras qui se présente sous forme de polymère solide à température ambiante ayant un poids moléculaire moyen en poids de 50 000 à 1 000 000. La gomme est souvent vendue en dispersion dans un solvant organique, du type huile de silicone.

La nature et la quantité des gommes ou cires sont fonction des propriétés mécaniques et des textures recherchées. A titre indicatif, la cire peut représenter de 0,01 à 50 %, de préférence de 2 à 40 %, et mieux de 5 à 30 % du poids total de chaque composition du produit.

En particulier, la cire peut être présente sous forme d'émulsion cire(s)-dans-eau.

La cire peut être présente dans chaque composition en une teneur allant de 0,01 % à 50 % en poids, en particulier de 0,1 % à 30 % en poids, et notamment de 0,2 % à 20 % en poids, par rapport au poids total de la composition.

Préparation

Chaque composition peut être obtenue selon les procédés de préparation classiquement utilisés en cosmétique ou en dermatologie.

Le produit peut se présenter sous une forme extrudée, compactée ou coulée en stick ou en coupelle. Les compositions du produit peuvent être coextrudées.

Le produit peut constituer, entre autres produits de maquillage, un produit à appliquer sur les lèvres, par exemple un brillant à lèvres, un rouge à lèvres, un baume à lèvres, un fard à joues, un fond de teint, un produit de contour des yeux, un eye-liner, une ombre à paupières, une base de maquillage, un produit de maquillage du corps ou des cheveux.

Conditionnement et modes d'application

On a représenté à titre d'exemple à la figure 1 un stick S qui comporte deux blocs A et B s'étendant sur toute la longueur du stick.

Chaque bloc A ou B occupe par exemple la moitié du stick et ces deux blocs sont par exemple accolés selon le diamètre du stick.

Le stick S peut être conditionné dans un dispositif de conditionnement et de distribution comportant par exemple un corps C dans lequel le stick est reçu au moins partiellement, comme illustré à la figure 2, et un mécanisme d'entraînement M permettant de déplacer le stick relativement au corps C.

Le dispositif de conditionnement et de distribution peut être de tout type adapté au conditionnement d'un stick et le mécanisme d'entraînement M comporte par exemple une molette rotative en partie inférieure.

Les deux blocs A et B peuvent être de même proportion ou non au sein du stick.

L'un des blocs peut être entouré par l'autre bloc, comme illustré à la figure 3.

Les deux blocs A et B peuvent chacun présenter un écart de couleur $\Delta E$ entre la couleur au sein du stick et la couleur après application sur les matières kératiniques.

Seul l'un des blocs A ou B peut présenter cette caractéristique, l'autre bloc ne présentant pas par exemple cette propriété.

Les deux blocs A et B peuvent présenter ou non sensiblement la même couleur au sein du stick, par exemple la couleur blanche.

Le stick peut comporter, le cas échéant, plus de deux blocs, par exemple trois blocs comme illustré à la figure 4. Le troisième bloc E peut ou non présenter un écart de couleur entre la couleur au sein du stick, dans la masse, et la couleur après application sur les matières kératiniques.

Le stick peut être réalisé par co-extrusion, coulage ou compactage des compositions destinées à former les différents blocs.

Dans l'exemple de la figure 5, le produit comporte deux blocs A et B coulés ou compactés dans une coupelle.

Dans une variante non illustrée, les blocs de composition sont contenus dans une gaine de manière à former un crayon.

Chaque bloc A ou B peut avoir une section transversale constante sur toute la largeur du produit. En variante, l'un des blocs au moins peut présenter une section transversale variable le long de la longueur du produit.

L'un des blocs peut présenter une section transversale circulaire.

Un bloc peut présenter, en coupe longitudinale, une forme en biseau s'élargissant vers une extrémité longitudinale tandis que l'autre bloc présente également une forme en biseau, s'élargissant dans le sens inverse, comme illustré en coupe longitudinale à la figure 6.

Procédé de présentation et présentoir

L'invention a encore pour objet, selon un autre de ses aspects, indépendamment ou en combinaison avec ce qui précède, un procédé de présentation d'une composition cosmétique réalisée conformément à l'invention, comportant les étapes consistant à :

- éclairer au moins une première région d'un échantillon de composition avec un éclairage diffus de façon à faire apparaître la couleur dans la masse,
- éclairer au moins une deuxième région d'un échantillon de composition avec un éclairage directionnel de façon à faire apparaître la couleur interférentielle.

Le produit peut être éclairé de façon à faire apparaître simultanément toutes les couleurs des compositions après application.

Les première et deuxième régions peuvent être des régions différentes d'un même échantillon de composition.

En variante, les première et deuxième régions appartiennent à des échantillons différents.

En variante encore, les première et deuxième régions correspondent à une même région et les éclairages diffus et directionnel sont appliqués alternativement.

L'invention a encore pour objet, selon un autre de ses aspects, un présentoir comportant :

- une source d'éclairage directionnel,
- une source d'éclairage diffus,
- un support pour permettre d'exposer au moins un échantillon de composition à la source d'éclairage diffus et au moins un échantillon de composition à la source d'éclairage directionnel.

Procédé de maquillage

L'invention a encore pour objet un procédé de maquillage des matières kératiniques au moyen d'un produit selon l'invention.
Il peut s'agir du maquillage de la peau, des lèvres ou cheveux.
Le maquillage peut par exemple s'effectuer sans mélange des compositions à l'application.
On peut par exemple déplacer un stick par un mouvement de translation de façon à créer deux traces colorées ayant des couleurs différentes et correspondant chacune au dépôt de la composition de l'un des blocs.
L'application peut encore s'effectuer de manière à provoquer un mélange ou une superposition des compositions des blocs lors de l'application.
Dans une variante où les blocs de composition sont contenus dans une coupelle, chacune des compositions est par exemple prélevée au moyen d'un applicateur ou du doigt.

Exemple proposé pour le stick multicolore

Les teneurs indiquées sont massiques.

Exemple 24 : Rouge à lèvres

Première composition

| | |
|---|---|
| Tri-mellitate de tri-decyle | 11 |
| Lanoline liquide | 10 |
| Malate de d'iso-stearyle | 12 |
| Lanoline acetylée | 10 |
| Triglycerides d'acides laurique/palmitique/cetylique/stearique (50/20/10/10) | 5 |
| Cire microcrystalline (C20-C60) | 3 |
| Lanolate d'isopropyle protégé | 9 |
| Octyl-2-decanol | 15 |
| Phényl trimethylsiloxy trisiloxane (VISCOSITE: 20 CST - PM: 372) | 4 |
| Cire de polyethylene (PM :500) | 8 |
| Pigment interferentiel multicouche * | 10 |
| Pigment à réflexion métallique** | 3 |
| * TIMIRON SILK RED de la société MERCK<br>** *METASHINE ME 2040 PS* de la société NIPPON SHEET GLASS | |

Deuxième composition

| | |
|---|---|
| Tri-mellitate de tri-decyle | 11 |
| Lanoline liquide | 10 |
| Malate de d'iso-stearyle | 12 |
| Lanoline acetylée | 10 |
| Triglycerides d'acides laurique/palmitique/cetylique/stearique (50/20/10/10) | 5 |
| Cire microcrystalline (C20-C60) | 3 |
| Lanolate d'isopropyle protégé | 9 |
| Octyl-2-decanol | 15 |
| Phényl trimethylsiloxy trisiloxane (VISCOSITE: 20 CST - PM: 372) | 4 |

(suite)

| | |
|---|---|
| Cire de polyethylene (PM :500) | 8 |
| Pigment interferentiel multicouche * | 10 |
| Pigment à réflexion métallique** | 3 |
| * TIMIRON SILK BLUE de la société MERCK<br>** *METASHINE ME 2040 PS* de la société NIPPON SHEET GLASS | |

Les deux compositions sont coextrudées pour former deux blocs produisant à l'application des couleurs différentes.

La première composition, rouge, occupe par exemple le centre du stick et la deuxième composition, bleue, sa périphérie, les deux blocs étant concentriques. Du violet peut apparaître à l'application, au centre, délimité par des bordures bleues.

Dans l'exemple 24, le pigment à reflet métallique pourrait encore être remplacé par un pigment diffractant ou un agent Xchrome.

Bien entendu, l'invention n'est pas limitée aux exemples qui viennent d'être donnés. L'expression « comportant un » est synonyme de « comportant au moins un » et « compris entre » s'entend bornes incluses.

## Revendications

**1.** Composition cosmétique comportant, dans un milieu cosmétiquement acceptable, au moins un pigment interférentiel multicouche et au moins l'un d'un agent de coloration sensible à au moins un stimulus extérieur, d'un pigment réfléchissant à reflet métallique et d'un pigment diffractant, la couvrance de la composition étant supérieure ou égale à 25, mieux 30, le pigment interférentiel multicouche étant en une teneur conférant à la composition un écart de couleur $\Delta E$ supérieur ou égale à 5 entre la couleur dans la masse et la couleur après application.

**2.** Composition selon la revendication 1, comportant un agent de coloration sensible à au moins un stimulus extérieur.

**3.** Composition selon la revendication 1, comportant un pigment réfléchissant à reflet métallique.

**4.** Composition selon la revendication 1, comportant un pigment diffractant.

**5.** Composition selon la revendication 1 ou 2, l'agent de coloration sensible à au moins un stimulus extérieur étant apte à prendre dans un état une couleur de longueur d'onde dominante éloignée de $\Delta\lambda$ de moins de 30 nm de la longueur d'onde dominante de la composition dans la masse.

**6.** Composition selon la revendication 1 ou 2, l'agent de coloration sensible à au moins un stimulus extérieur étant apte à prendre dans un état une couleur de longueur d'onde dominante éloignée de $\Delta\lambda$ de moins de 30 nm de la longueur d'onde dominante de la composition après application sur des matières kératiniques

**7.** Composition selon l'une des revendications 1 et 2, l'agent de coloration sensible à au moins un stimulus extérieur étant incolore dans un état.

**8.** Composition selon la revendication 7, l'état correspondant à la présence du stimulus.

**9.** Composition selon la revendication 7, l'état correspondant à l'absence de stimulus.

**10.** Composition selon l'une des revendications 8 ou 9, l'état se rencontrant dans la masse de la composition, avant l'application sur des matières kératiniques.

**11.** Composition selon l'une quelconque des revendications 8 ou 9, l'état se rencontrant après l'application sur des matières kératiniques.

**12.** Composition selon l'une quelconque des revendications 1, 2 et 5 à 11, l'agent de coloration sensible à au moins un stimulus extérieur étant en solution dans le milieu.

**13.** Composition selon l'une quelconque des revendications 1, 2 et 5 à 11, l'agent de coloration sensible à au moins un stimulus extérieur étant dispersé sous forme particulaire dans le milieu.

**14.** Composition selon l'une quelconque des revendications 1, 2 et 5 à 13, l'agent de coloration sensible à au moins un stimulus extérieur étant un agent photochrome.

**15.** Composition selon l'une quelconque des revendications 1, 2 et 5 à 13, l'agent de coloration sensible à au moins un stimulus extérieur étant un agent thermochrome.

**16.** Composition selon l'une quelconque des revendications 1, 2 et 5 à 13, l'agent de coloration sensible à au moins un stimulus extérieur étant un agent solvatochrome.

**17.** Composition selon l'une quelconque des revendications 1, 2 et 5 à 13, l'agent de coloration sensible à au moins un stimulus extérieur étant piézochrome ou tribochrome ou mécanoluminescent.

**18.** Composition selon l'une quelconque des revendications précédentes, le ratio $m_1/m_2$ entre la teneur massique $m_1$ en pigment interférentiel multicouche et la teneur totale $m_2$ en agent de coloration sensible à un stimulus extérieur ou en pigment réfléchissant à reflet métallique ou en pigment diffractant allant de 0,1 à 1,5.

**19.** Composition selon l'une quelconque des revendications précédentes, le pigment interférentiel multicouche comportant un substrat de silice, mica ou verre, alumine ou métal.

**20.** Composition selon l'une quelconque des revendications 1 à 19, la composition étant anhydre.

**21.** Composition selon l'une quelconque des revendications 1 à 19, la composition étant aqueuse.

**22.** Composition selon l'une quelconque des revendications 1 à 21, la composition étant non pulvérulente et la teneur massique totale en pigment interférentiel multicouche allant de 7 à 20 %, mieux 8 et 15 %.

**23.** Composition selon la revendication 22, la composition étant liquide.

**24.** Composition selon la revendication 22, la composition étant sous forme de stick.

**25.** Composition selon l'une quelconque des revendications 1 à 21, la composition étant pulvérulente et la teneur massique totale en pigment interférentiel multicouche allant de 40 à 95 %.

**26.** Composition selon l'une quelconque des revendications 1 à 25, la couleur de la composition dans la masse étant blanche, lorsque la composition est non exposée au stimulus.

**27.** Composition selon la revendication 26, la composition dans la masse ayant un indice de blancheur supérieur ou égal à 40.

**28.** Composition selon l'une quelconque des revendications 1 à 27, la composition ne comportant pas d'autre agent de coloration que le ou les pigments interférentiels multicouche, et

- le ou les agents de coloration sensibles à un stimulus extérieur, ou
- le ou les pigments réfléchissant à reflet métallique, ou
- le ou les pigments diffractants.

**29.** Composition selon l'une quelconque des revendications 1 à 28, le pigment interférentiel multicouche comportant au moins quatre couches.

**30.** Composition selon l'une quelconque des revendications 1 à 29, le pigment interférentiel multicouche comportant un substrat en une matière transparente.

**31.** Composition selon l'une quelconque des revendications 1 à 30, le pigment interférentiel multicouche comportant un substrat en silice, mica ou verre.

**32.** Composition selon l'une quelconque des revendications précédentes, la couleur produite par le pigment interférentiel multicouche n'étant pas de longueur d'onde dominante comprise entre 580 et 650 nm.

**33.** Composition selon l'une quelconque des revendications 1 à 32, ne comportant qu'un seul pigment interférentiel multicouche.

**34.** Composition selon l'une quelconque des revendications 1 à 32, comportant au moins deux pigments interférentiels multicouche.

**35.** Composition selon la revendication 34, les deux pigments interférentiels multicouche ayant des couches constituées des mêmes matériaux mais au moins une couche d'un pigment ayant une épaisseur différente d'une couche correspondante de l'autre pigment.

**36.** Composition cosmétique non pulvérulente comportant, dans un milieu cosmétiquement acceptable :

- entre 7 et 20 % en masse, au total, d'un ou plusieurs pigments interférentiels multicouche,
- entre 0,1 et 10 % en masse, au total, d'un ou plusieurs agents de coloration sensible à au moins un stimulus extérieur, ou d'un ou plusieurs pigments réfléchissants à reflet métallique ou d'un ou plusieurs pigments diffractants.

**37.** Composition selon la revendication 36, la composition étant sous forme de stick.

**38.** Composition selon la revendication 36, la composition étant liquide.

**39.** Composition selon l'une des revendications 36 à 38, la teneur totale en pigment(s) interférentiel(s) multicouche allant de 8 à 15 %.

**40.** Composition cosmétique pulvérulente comportant, dans un milieu cosmétiquement acceptable :

- entre 40 et 95 % en masse, au total, d'un ou plusieurs pigments interférentiels multicouche,
- entre 0,1 et 60 % en masse, au total, d'un ou plusieurs agents de coloration sensibles à au moins un stimulus extérieur, ou d'un ou plusieurs pigments réfléchissants à reflet métallique ou d'un ou plusieurs pigments diffractants.

**41.** Composition selon la revendication 40, la composition comportant entre 55 et 65 % en masse de pigment(s) interférentiel(s) multicouche.

**42.** Composition selon l'une quelconque des revendications 40 et 41, la composition comportant entre 1 et 50 % en masse au total d'agent(s) de coloration sensible à au moins un stimulus extérieur ou de pigments réfléchissants à reflet métallique ou de pigments diffractants.

**43.** Composition selon l'une quelconque des revendications 1 à 42, comportant moins de 0,5% en masse de pigments générant une couleur par absorption.

**44.** Composition selon la revendication 43, les compositions contenant moins de 0,5% de pigments à base d'oxyde de fer.

**45.** Composition selon l'une quelconque des revendications précédentes, le pigment interférentiel multicouche étant dépourvu de couche colorée.

**46.** Composition selon l'une quelconque des revendications précédentes, le pigment interférentiel ne comportant, sur le substrat, qu'une ou plusieurs couches choisies parmi Ti02, Zr02, Sn02, Si02.

**47.** Composition selon l'une quelconque des revendications précédentes, le pigment interférentiel multicouche étant dépourvu de Fe203.

**48.** Composition selon l'une quelconque des revendications précédentes, la teneur massique en pigment interférentiel multicouche étant supérieure à 7%.

**49.** Composition selon la revendication 3, le pigment réfléchissant à reflet métallique comportant une couche d'un métal ou alliage métallique en surface.

**50.** Composition selon la revendication 3, le pigment réfléchissant à reflet métallique comportant une couche d'un oxyde métallique en surface.

**51.** Composition selon la revendication 3, le pigment réfléchissant à reflet métallique présentant une forme plaquettaire.

**52.** Composition selon la revendication 3, le pigment réfléchissant à reflet métallique présentant une forme sensiblement sphérique.

**53.** Composition selon la revendication 3, la taille du pigment réfléchissant à reflet métallique étant comprise entre 10 et 500 μm.

**54.** Composition selon la revendication 3, la taille du pigment réfléchissant à reflet métallique étant comprise entre 10 et 150 μm, mieux entre 40 et 150 μm.

**55.** Composition selon la revendication 3, le ratio m1/m2 entre la teneur massique m1 en pigment interférentiel multicouche et la teneur m2 en pigment réfléchissant à reflet métallique allant de 0,1 à 1,5.

**56.** Composition selon la revendication 3, le pigment interférentiel multicouche et le pigment réfléchissant à réflexion métallique présentant un substrat dans le même matériau.

**57.** Composition selon la revendication 4, la teneur massique en pigment diffractant allant de 0,1 à 60.

**58.** Composition selon la revendication 4, le pigment diffractant présentant une structure multicouche.

**59.** Composition selon la revendication 4, le pigment interférentiel multicouche et le pigment diffractant présentant un substrat dans le même matériau.

**60.** Ensemble de conditionnement comportant :

- une composition telle que définie dans l'une quelconque des revendications précédentes,
- un moyen permettant de renseigner l'utilisateur sur la couleur de la composition après l'application sur des matières kératiniques.

**61.** Procédé de maquillage des matières kératiniques, comportant l'application sur les matières kératiniques d'une composition selon l'une quelconque des revendications 1 à 59.

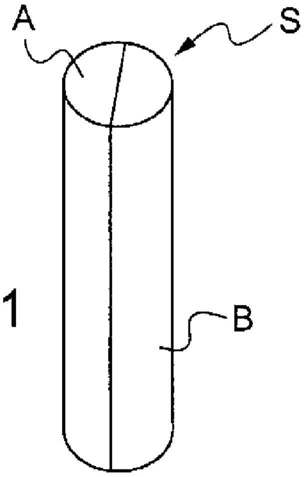


Fig.1

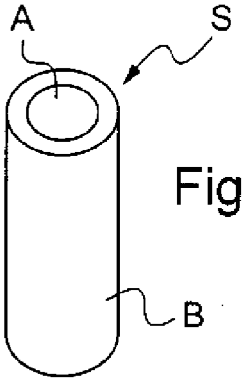


Fig.3

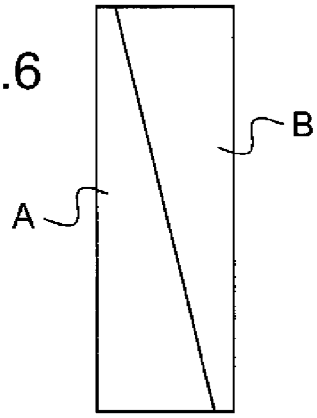


Fig.6

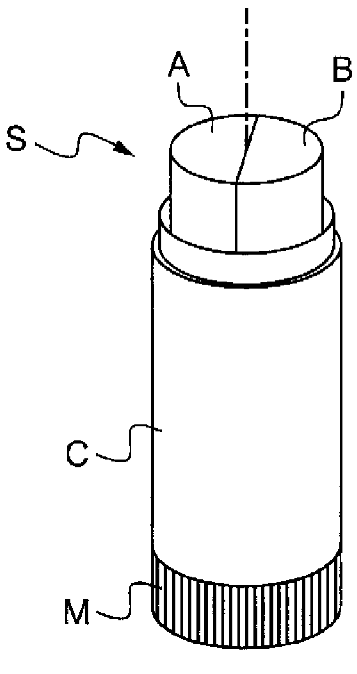


Fig.2

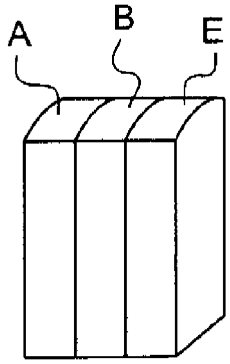


Fig.4

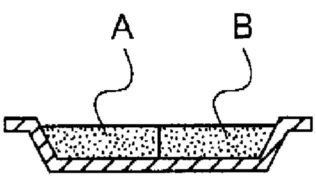


Fig.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20040228818 A
- WO 9426729 A
- US 6071632 A
- US 20020015965 A
- WO 09016429 A
- US 6280655 B
- EP 1318184 A
- JP 2002194349 A
- JP 2004059746 A
- US 6117574 A
- JP 2004043656 A
- US 20030031870 A
- EP 1172091 A


### Littérature non-brevet citée dans la description


- **D'ALBERTO ARGOITIA ; MATT WITZMAN.** Pigments Exhibiting Diffractive Effects. *Society of Vacuum coaters, 45th Annual Technical Conference, Proceedings 2002,* 2002
- International Cosmetic Ingredient Dictionnary and Handbook. The Cosmetic, Toiletry, and Fragrance Association, 1997, 371-386524-528